# EUROPEAN PATENT APPLICATION

(11) **EP 4 269 589 A1**
(43) Date of publication of application: **01.11.2023**
(21) Application number: 21910614.3
(22) Date of filing: 16.12.2021
(51) Int. Cl.: C12N 15/31, A01G 7/06, A01N 63/20, A01P 21/00, C12N 1/20, C12N 1/21, C12N 15/54

(54) **METHOD FOR PRODUCING PLANT ACIDIC INVERTASE ACTIVATOR, PLANT ACIDIC INVERTASE ACTIVATOR, AND PLANT ACIDIC INVERTASE ACTIVATION METHOD**

(30) Priority: 25.12.2020 JP 2020217002
(71) Applicant: Panasonic Intellectual Property Management Co., Ltd., Kadoma-shi, Osaka 571-0057 (JP)
(72) Inventor: KOJIMA, Seiji, Kadoma-shi, Osaka 571-0057 (JP)
(74) Representative: Novagraaf International SA
(86) International application number: PCT/JP2021/046626
(87) International publication number: WO 2022/138466

(57) **Abstract**

A plant acidic invertase activator production method includes: preparing a modified cyanobacterium in which a function of a protein involved in binding between an outer membrane (5) and a cell wall (4) of cyanobacterium is suppressed or lost (step S01); and causing the modified cyanobacteria to secrete a secretion involved in activating an acidic invertase of a plant (step S02).

## Description

### [Technical Field]

The present disclosure relates to a production method for a plant acidic invertase activator, which is a natural metabolite having an effect of activating acidic invertase of a plant, a plant acidic invertase activator, and a plant acidic invertase activation method.

### [Background Art]

With the demand for increased food production associated with a growing world population, there is a need for the development of techniques for efficiently producing crops in limited cultivated land. Approaches aimed at increasing yields of crops are broadly classified into a method of controlling environmental factors such as light, temperature and humidity, and soil components to conditions suitable for the growth of crops (i.e., a method of performing environmental control), and a method of artificially controlling the cellular physiology of plants by use of a gene recombination technique or by the addition of physiologically active substances or the like to promote their growth (i.e., a method of controlling cellular physiology).

The activity control of invertase by gene recombination is known as a method of controlling cellular physiology (Non Patent Literature (NPL) 1). Invertase is an enzyme that degrades sucrose (so-called table sugar) into glucose and fructose, and is deeply involved in the translocation, distribution, and accumulation of sucrose formed by photosynthesis performed in leaves to each organ of the plant. The invertase is broadly classified into neutral invertase and acidic invertase, depending on the optimum pH thereof. The acidic invertase includes two types: cell wall invertase which is localized in the cell wall, and vacuolar invertase which is localized in the vacuole. It is known that, particularly, the activity of acidic invertase among the invertases is closely related to crop yields. For example, NPL 2 discloses that the cotton fiber production of cotton is enhanced by activating vacuolar invertase by a gene recombination technique. Also, NPL 3 discloses that vacuolar invertase activity is essential for the growth of rice stalks. For example, NPL 4 and 5 disclose that soybean yields and corn yields are increased by activating cell wall invertase of corn and soybean by a gene recombination technique; and sugar contents of their respective grains are increased. From these, the development of techniques of enhancing agricultural crop productivity by artificially activating acidic invertase is expected.

### [Citation List]

### [Non Patent Literature]

[NPL 1] Roitsch, T. and M. C. Gonzalez (2004), "Function and regulation of plant invertases: sweet sensations", Trends in Plant Science 9(12): 606-613
[NPL 2] Wang, L., et al. (2010), "Evidence that high activity of vacuolar invertase is required for cotton fiber and Arabidopsis root elongation through osmotic dependent and independent pathways, respectively", Plant Physiology, 154(2): 744-756
[NPL 3] Morey, S. R., et al. (2018), "Genetic Evidence for the Role of a Rice Vacuolar Invertase as a Molecular Sink Strength Determinant", Rice 11
[NPL 4] Tang, X., et al. (2017), "Suppression of extracellular invertase inhibitor gene expression improves seed weight in soybean (Glycine max)", Journal of Experimental Botany 68(3): 469-482 [NPL 5] Li, B., et al. (2013), "Constitutive expression of cell wall invertase genes increases grain yield and starch content in maize", Plant Biotechnology Journal 11(9): 1080-1091

### [Summary of Invention]

### [Technical Problem]

However, approaches of activating acidic invertase are limited to approaches based on a gene recombination technique at present and therefore, are disadvantageously difficult to put into practical application. This is because laws and regulations on the gene recombination technique throughout the world as well as producers' and consumers' emotional feelings of rejection against the application (hereinafter, also referred to as use) of the gene recombination technique to plants become a major barrier to the social implementation of the techniques described above. Hence, there is a need for approaches of activating acidic invertase in crops by stimulation from the outside (e.g., the addition of some substance to plants from the outside) without the use of the gene recombination technique.

In view of this, the present disclosure provides a method for conveniently and efficiently producing a plant acidic invertase activating substance that activates the acidic invertase of a plant. The present disclosure also provides a plant acidic invertase activator that can efficiently activate the acidic invertase of a plant, and a plant acidic invertase activation method.

### [Solution to Problem]

A plant acidic invertase activator production method according to an aspect of the present disclosure includes: preparing a modified cyanobacterium in which a function of a protein involved in binding between an outer membrane and a cell wall of cyanobacterium is suppressed or lost; and causing the modified cyanobacteria to secrete a secretion involved in activating an acidic invertase of a plant.

### [Advantageous Effects of Invention]

The plant acidic invertase activator production method of the present disclosure can conveniently and efficiently produce a plant acidic invertase activator that activates the acidic invertase of a plant. Furthermore, the plant acidic invertase activator of the present disclosure can effectively activate the acidic invertase of a plant. In addition, the plant acidic invertase activation method of the present disclosure can effectively activate the acidic invertase of a plant by using the plant acidic invertase activator of the present disclosure on a plant.

### [Brief Description of Drawings]

[FIG. 1]

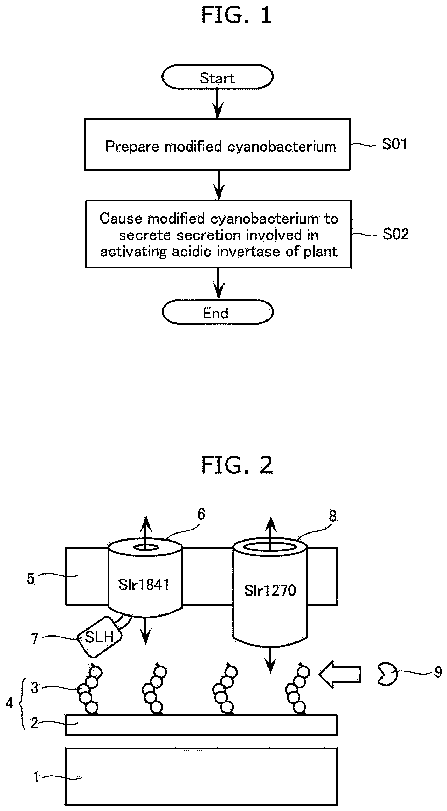

   FIG. 1 is a flow chart illustrating one example of a plant acidic invertase activator production method according to an embodiment.
[FIG. 2]
   FIG. 2 is a diagram schematically illustrating a cell surface of a cyanobacterium.
[FIG. 3]

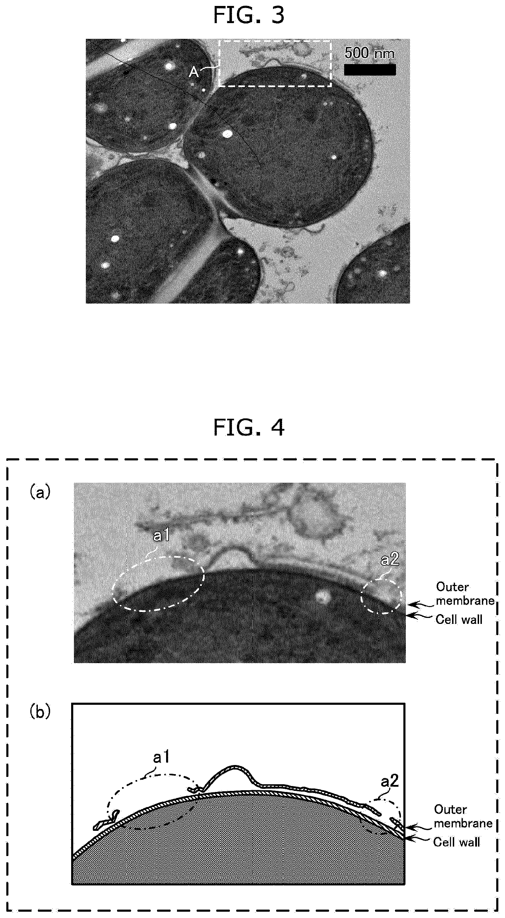

   FIG. 3 is a transmission electron microscope image of an ultrathin section of a modified cyanobacterium of Example 1.
[FIG. 4]
   FIG. 4 is an enlarged image of broken line region A of FIG. 3.
[FIG. 5]

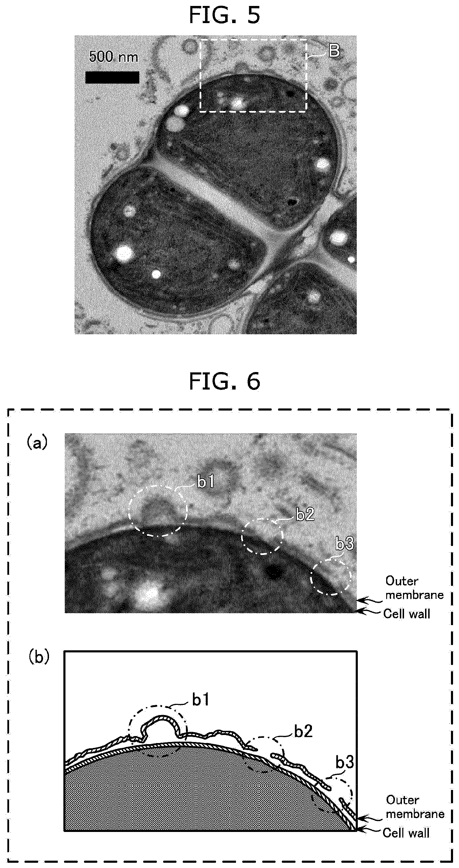

   FIG. 5 is a transmission electron microscope image of an ultrathin section of a modified cyanobacterium of Example 2.
[FIG. 6]
   FIG. 6 is an enlarged image of broken line region B of FIG. 5
[FIG. 7]

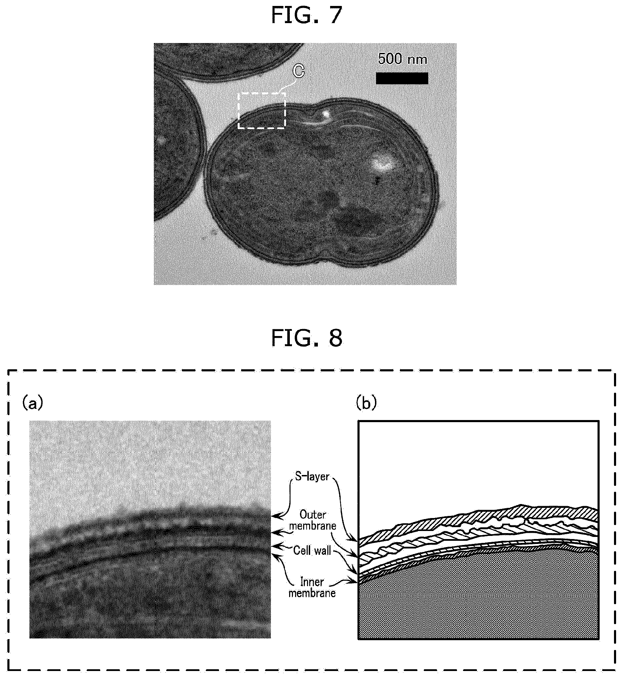

   FIG. 7 is a transmission electron microscope image of an ultrathin section of a modified cyanobacterium of Comparative Example 1.
[FIG. 8]
   FIG. 8 is an enlarged view of broken line region C of FIG. 7.
[FIG. 9]

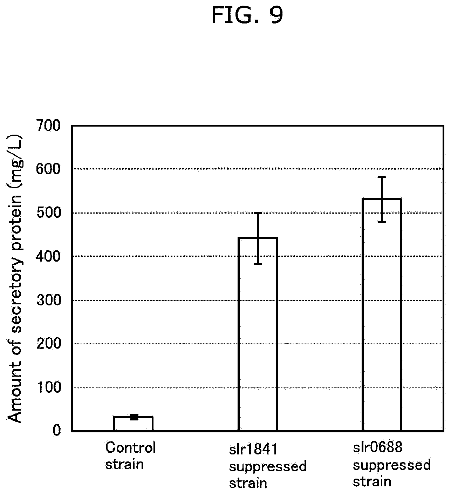

   FIG. 9 is a graph illustrating the amount of protein (n = 3, error bar = SD) in the culture supernatant of a modified cyanobacterium in Example 1, Example 2, and Comparative Example 1.
[FIG. 10]

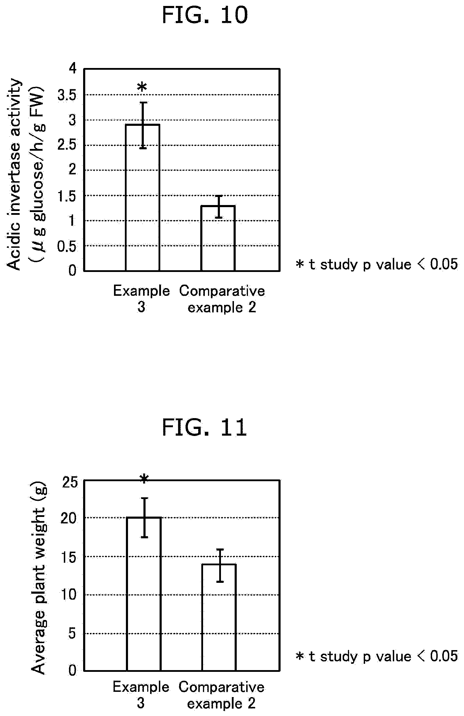

   FIG. 10 is a graph illustrating a mean of the acidic invertase activity of spinach cultivated in Example 3 and Comparative Example 2.
[FIG. 11]
   FIG. 11 is a graph illustrating a mean of the dry weight of shoot per plant of spinach cultivated in Example 3 and Comparative Example 2.
[FIG. 12]

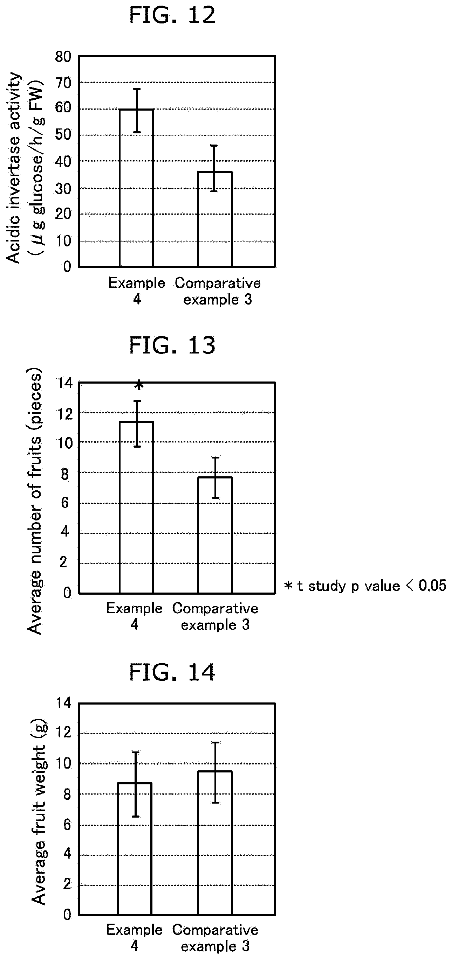

   FIG. 12 is a graph illustrating a mean of the acidic invertase activity of strawberry cultivated in Example 4 and Comparative Example 3.
[FIG. 13]
   FIG. 13 is a graph illustrating an average number of fruits per plant of strawberry cultivated in Example 4 and Comparative Example 3.
[FIG. 14]
   FIG. 14 is a graph illustrating an average fruit weight per plant of strawberry cultivated in Example 4 and Comparative Example 3.
[FIG. 15]

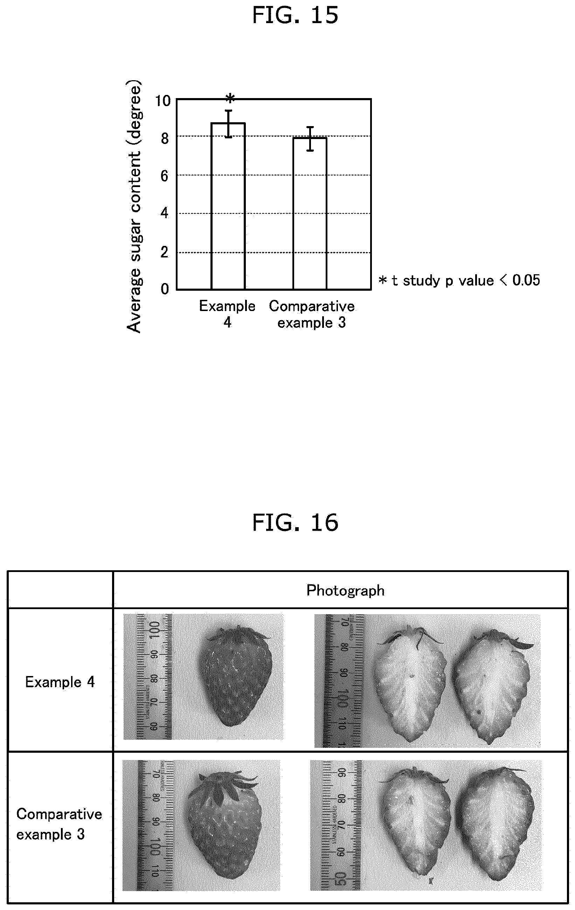

   FIG. 15 is a graph illustrating an average sugar content per plant of strawberry cultivated in Example 4 and Comparative Example 3.
[FIG. 16]
   FIG. 16 is a diagram illustrating the states of respective typical fruits in Example 4 and Comparative Example 3.

### [Description of Embodiments]

### (Underlying knowledge forming basis of present disclosure)

As mentioned in the Background Art, there is a need for techniques for efficiently producing agricultural crops in limited cultivated lands. Techniques of activating plant acidic invertase are expected as approaches therefor.

The following conventional techniques are disclosed as techniques of activating plant acidic invertase.

For example, NPL 2 has reported that the elongation of cotton fibers is promoted by highly expressing cotton vacuolar invertase gene using 35S promoter.

For example, NPL 4 has reported that cell wall invertase is activated by inhibiting the expression of a gene that inhibits the cell wall invertase activity of soybean by RNA (ribonucleic acid) interference. Specifically, it has been reported that this technique used for soybean increases a weight per grain of soybean, increases a harvest weight per plant, and further increases a sugar content per grain of soybean.

For example, NPL 5 has reported that the yield of corn is increased by highly expressing corn cell wall invertase gene using 35S promoter. It has also been reported that a sugar content per grain is increased.

However, approaches of activating plant acidic invertase are limited to approaches based on a gene recombination technique at present and therefore, are disadvantageously difficult to put into practical application. This is because laws and regulations on the gene recombination technique throughout the world as well as producers' and consumers' emotional feelings of rejection against the use of the gene recombination technique in plants become a major barrier to the social implementation of the techniques described above. Hence, there is a strong need for approaches of activating acidic invertase in crops by the addition of some substance to plants from the outside without the use of the gene recombination technique. Furthermore, there is a demand for the exploitation of naturally derived substances with less environmental load in application because environmental consciousness such as the prevention of global warming and reduction in environmental load has been expanded in recent years. Among other, there is a demand, particularly, for the application of naturally derived substances with less consumption of fossil energy in the production of the substances and with much less environmental load.

The present inventors have focused on cyanobacterium as a microbe for use in the production of naturally-derived substances that contribute to the enhancement of agricultural crop productivity. Cyanobacterium (also called blue-green bacterium or blue-green alga), a group of eubacterium, produces oxygen by splitting water through photosynthesis, and fixes CO₂ in air. Cyanobacterium can also fix nitrogen (N₂) in air, depending on its species. Thus, cyanobacterium can obtain a large part of starting materials (i.e., nutrients) and energy necessary for bacterial cell growth from air, water, and light and can therefore be cultured by a convenient process using an inexpensive starting material.

Rapid growth and high light use efficiency are known as characteristics of cyanobacterium. In addition, its genetic manipulation is easier than that of other eukaryotic alga species. Therefore, bio-manufacturing that utilizes cyanobacterium among the photosynthetic microbes is under active research and development. For example, the production of fuels such as ethanol, isobutanol, alkanes, and fatty acids (PTL 1: Japanese Patent No. 6341676) has been reported as examples of bio-manufacturing using cyanobacterium. The production of substances serving as nutrient sources for organisms is also under research and development. For example, since protein can be synthesized only by living organisms, there is a need for the development of techniques of conveniently and efficiently producing protein. The exploitation of cyanobacterium which can utilize light energy and CO₂ in the air is expected as an organism species for use in such techniques, and is under active research and development (NPL 6: Jie Zhou et al., "Discovery of a super-strong promoter enable efficient production of heterologous proteins in cyanobacteria", Scientific Reports, Nature Research, 2014, Vol. 4, Article No. 4500).

For example, use of the technique described in NPL 6 to arbitrarily modify a cyanobacterium gene enables a desired compound and protein to be produced within the cell of cyanobacterium (hereinafter, also referred to as within the bacterial cell). However, since the intracellularly produced desired compound and protein of cyanobacterium are difficult to secrete to the outside of the cell, it is necessary to disrupt the cell of cyanobacterium and extract the intracellularly produced desired compound and protein.

Accordingly, the present inventors have found that the desired compound and protein produced within the bacterial cell of cyanobacterium and metabolites within the bacterial cell are easily secreted to the outside of the bacterial cell by partially detaching the outer membrane which surrounds the cell wall of cyanobacterium from the cell wall. During this process, the present inventors have also found that a secretion of cyanobacterium has an acidic invertase activating effect on a plurality of crop species. Accordingly, a substance that activates plant acidic invertase (i.e., a plant acidic invertase activating substance) secreted to the outside of the bacterial cell can be efficiently retrieved without disrupting the bacterial cell of the cyanobacterium. Furthermore, the physiological activity of the plant acidic invertase activating substance is less likely to be impaired because operations such as extraction are unnecessary. Therefore, a plant acidic invertase activator containing the secretion can effectively activate the acidic invertase of the plant.

Thus, the plant acidic invertase activator production method of the present disclosure can conveniently and efficiently produce a plant acidic invertase activator containing a substance having an acidic invertase activating effect on a plurality of crop species. The plant acidic invertase activator of the present disclosure can effectively activate the acidic invertase of a plant. The plant acidic invertase activation method of the present disclosure can effectively activate the acidic invertase of a plant by using the plant acidic invertase activator of the present disclosure in plants.

### (Outline of present disclosure)

An outline of an aspect of the present disclosure is as described below.

A plant acidic invertase activator production method according to an aspect of the present disclosure includes: preparing a modified cyanobacterium in which a function of a protein involved in binding between an outer membrane and a cell wall of cyanobacterium is suppressed or lost; and causing the modified cyanobacteria to secrete a secretion involved in activating an acidic invertase of a plant.

As a result, the binding (e.g., binding level and binding force) between the cell wall and the outer membrane is partially reduced in the modified cyanobacterium. This facilitates partially detaching the outer membrane from the cell wall. Hence, protein and metabolites produced within the bacterial cell (hereinafter, also referred to as intra-bacterial cell produced substances) easily leak out to the outside of the outer membrane, i.e., the outside of the bacterial cell. This facilitates secreting protein and metabolites produced within the bacterial cell of the modified cyanobacterium to the outside of the bacterial cell and therefore eliminates the need of extraction treatment of the intra-bacterial cell produced substances, such as the disruption of the bacterial cell. Hence, a plant acidic invertase activator containing a secretion of the modified cyanobacterium can be produced conveniently and efficiently. The intra-bacterial cell produced substances are less susceptible to reduction in physiological activity and yield because the extraction treatment of the intra-bacterial cell produced substances is unnecessary. Hence, a substance involved in activating acidic invertase of a plant (i.e., a plant acidic invertase activating substance) among the intra-bacterial cell produced substances of the modified cyanobacterium is also less susceptible to reduction in physiological activity and yield. As a result, the secretion of the modified cyanobacterium has an improved effect involved in activating of acidic invertase of a plant (hereinafter, also referred to as a plant acidic invertase activating effect). The intra-bacterial cell produced substances can be produced by repeatedly using the modified cyanobacterium even after retrieval of the intra-bacterial cell produced substances secreted to the outside of the bacterial cell because the extraction treatment of the intra-bacterial cell produced substances is unnecessary. This eliminates the need of providing a fresh modified cyanobacterium for each plant acidic invertase activator production. Thus, the plant acidic invertase activator production method according to an aspect of the present disclosure can conveniently and efficiently produce a plant acidic invertase activator.

For example, in the plant acidic invertase activator production method according to an aspect of the present disclosure, the protein involved in the binding between the outer membrane and the cell wall may be at least one of a surface layer homology (SLH) domain-containing outer membrane protein or a cell wall-pyruvic acid modifying enzyme.

As a result, in the modified cyanobacterium, for example, (i) a function of at least one of a SLH domain-containing outer membrane protein which binds to the cell wall and an enzyme that catalyzes reaction to modify a linked sugar chain on the surface of the cell wall with pyruvic acid (i.e., a cell wall-pyruvic acid modifying enzyme) is suppressed or lost, or (ii) the expression of at least one of the SLH domain-containing outer membrane protein or the cell wall-pyruvic acid modifying enzyme is suppressed. Hence, the binding (i.e., binding level and binding force) between the SLH domain of the SLH domain-containing outer membrane protein in the outer membrane and a covalently linked sugar chain on the surface of the cell wall is reduced. Accordingly, the outer membrane is easily detached from the cell wall at a site having the weakened binding between the outer membrane and the cell wall. As a result, in the modified cyanobacterium, intra-bacterial cell produced substances such as protein and metabolites produced within the bacterial cell easily leak out to the outside of the bacterial cell, as described above, because the outer membrane is easy to partially detach from the cell wall by the weakened binding between the outer membrane and the cell wall. As a result, the modified cyanobacterium has improved secretory productivity to secrete a plant acidic invertase activating substance produced within the bacterial cell to the outside of the bacterial cell. Thus, the plant acidic invertase activator production method according to an aspect of the present disclosure can efficiently produce a plant acidic invertase activator containing a plant acidic invertase activating substance because the modified cyanobacterium can efficiently secrete the plant acidic invertase activating substance.

For example, in the plant acidic invertase activator production method according to an aspect of the present disclosure, the SLH domain-containing outer membrane protein may be: Slr1841 having an amino acid sequence represented by SEQ ID NO: 1; NIES970_09470 having an amino acid sequence represented by SEQ ID NO: 2; Anacy_3458 having an amino acid sequence represented by SEQ ID NO: 3; or a protein having an amino acid sequence that is at least 50 percent identical to the amino acid sequence of any one of the SIr1841, the NIES970_09470, and the Anacy_3458.

As a result, in the modified cyanobacterium, for example, (i) the function of the SLH domain-containing outer membrane protein represented by any one of SEQ ID NOs: 1 to 3 or a protein having an amino acid sequence that is at least 50 percent identical to the amino acid sequence of any one of these SLH domain-containing outer membrane proteins is suppressed or lost, or (ii) the expression of the SLH domain-containing outer membrane protein represented by any one of SEQ ID NOs: 1 to 3 or a protein having an amino acid sequence that is at least 50 percent identical to the amino acid sequence of any one of these SLH domain-containing outer membrane proteins is suppressed. Hence, in the modified cyanobacterium, (i) the function of the SLH domain-containing outer membrane protein or a protein functionally equivalent to the SLH domain-containing outer membrane protein in the outer membrane is suppressed or lost, or (ii) the expression level of the SLH domain-containing outer membrane protein or a protein functionally equivalent to the SLH domain-containing outer membrane protein in the outer membrane is decreased. As a result, in the modified cyanobacterium, the binding level and binding force with which a binding domain (e.g., the SLH domain) for binding the outer membrane with the cell wall binds to the cell wall are reduced. This facilitates partially detaching the outer membrane from the cell wall. As a result, intra-bacterial cell produced substances easily leak out to the outside of the bacterial cell, so that a plant acidic invertase activating substance produced within the bacterial cell also easily leaks out to the outside of the bacterial cell. Thus, the plant acidic invertase activator production method according to an aspect of the present disclosure can efficiently produce a plant acidic invertase activator because the plant acidic invertase activating substance produced within the bacterial cell of the modified cyanobacterium easily leaks out to the outside of the bacterial cell.

For example, in the plant acidic invertase activator production method according to an aspect of the present disclosure, the cell wall-pyruvic acid modifying enzyme may be: Slr0688 having an amino acid sequence represented by SEQ ID NO: 4; Synpcc7942_1529 having an amino acid sequence represented by SEQ ID NO: 5; Anacy_1623 having an amino acid sequence represented by SEQ ID NO: 6; or a protein having an amino acid sequence that is at least 50 percent identical to the amino acid sequence of any one of the Slr0688, the Synpcc7942_1529, and the Anacy_1623.

As a result, in the modified cyanobacterium, for example, (i) the function of the cell wall-pyruvic acid modifying enzyme represented by any one of SEQ ID NOs: 4 to 6 or a protein having an amino acid sequence that is at least 50 percent identical to the amino acid sequence of any one of these cell wall-pyruvic acid modifying enzymes is suppressed or lost, or (ii) the expression of the cell wall-pyruvic acid modifying enzyme represented by any one of SEQ ID NOs: 4 to 6 or a protein having an amino acid sequence that is at least 50 percent identical to the amino acid sequence of any one of these cell wall-pyruvic acid modifying enzymes is suppressed. Hence, in the modified cyanobacterium, (i) the function of the cell wall-pyruvic acid modifying enzyme or a protein functionally equivalent to the enzyme is suppressed or lost, or ii) the expression level of the cell wall-pyruvic acid modifying enzyme or a protein functionally equivalent to the enzyme is decreased. A covalently linked sugar chain on the surface of the cell wall is thereby less susceptible to modification with pyruvic acid, so that binding level and binding force of the sugar chain of the cell wall that binds to the SLH domain of the SLH domain-containing outer membrane protein in the outer membrane are reduced. As a result, in the modified cyanobacterium, a covalently linked sugar chain on the surface of the cell wall is less susceptible to modification with pyruvic acid, so that binding force between the cell wall and the outer membrane is weakened. This facilitates partially detaching the outer membrane from the cell wall. As a result, intra-bacterial cell produced substances easily leak out to the outside of the bacterial cell, so that a plant acidic invertase activating substance produced within the bacterial cell also easily leaks out to the outside of the bacterial cell. Thus, the plant acidic invertase activator production method according to an aspect of the present disclosure can efficiently produce a plant acidic invertase activator because the plant acidic invertase activating substance produced within the bacterial cell of the modified cyanobacterium easily leaks out to the outside of the bacterial cell.

For example, in the plant acidic invertase activator production method according to an aspect of the present disclosure, a gene which causes expression of the protein involved in the binding between the outer membrane and the cell wall may be deleted or inactivated.

Accordingly, in the modified cyanobacterium, the expression of the protein involved in the binding between the cell wall and the outer membrane is suppressed, or the function of the protein is suppressed or lost. Therefore, the binding (i.e., binding level and binding force) between the cell wall and the outer membrane is partially reduced. As a result, in the modified cyanobacterium, the outer membrane is easy to partially detach from the cell wall, so that intra-bacterial cell produced substances such as protein and metabolites produced within the bacterial cell easily leak out to the outside of the outer membrane, i.e., the outside of the bacterial cell. Hence, the modified cyanobacterium has improved secretory productivity of a plant acidic invertase activating substance produced within the bacterial cell. This eliminates the need of extraction treatment of the intra-bacterial cell produced substances, such as the disruption of the bacterial cell. Therefore, the intra-bacterial cell produced substances are less susceptible to reduction in physiological activity and yield. Hence, a plant acidic invertase activating substance produced within the bacterial cell is also less susceptible to reduction in physiological activity and yield. Therefore, a plant acidic invertase activator having an improved plant acidic invertase activating effect can be produced. The plant acidic invertase activating substance can be produced by repeatedly using the modified cyanobacterium even after retrieval of the intra-bacterial cell produced substances because the extraction treatment of the intra-bacterial cell produced substances is unnecessary. This eliminates the need of providing a fresh modified cyanobacterium for each plant acidic invertase activator production. Thus, the plant acidic invertase activator production method according to an aspect of the present disclosure can conveniently and efficiently produce a plant acidic invertase activator.

For example, in the plant acidic invertase activator production method according to an aspect of the present disclosure, the gene which causes expression of the protein involved in the binding between the outer membrane and the cell wall may be at least one of a gene encoding an SLH domain-containing outer membrane protein or a gene encoding a cell wall-pyruvic acid modifying enzyme.

Accordingly, in the modified cyanobacterium, at least one of the gene encoding the SLH domain-containing outer membrane protein and the gene encoding the cell wall-pyruvic acid modifying enzyme is deleted or inactivated. Hence, in the modified cyanobacterium, for example, (i) the expression of at least one of the SLH domain-containing outer membrane protein or the cell wall-pyruvic acid modifying enzyme is suppressed, or (ii) the function of at least one of the SLH domain-containing outer membrane protein or the cell wall-pyruvic acid modifying enzyme is suppressed or lost. Hence, the binding (i.e., binding level and binding force) between the SLH domain of the SLH domain-containing outer membrane protein in the outer membrane and a covalently linked sugar chain on the surface of the cell wall is reduced. Accordingly, the outer membrane is easily detached from the cell wall at a site having the weakened binding between the outer membrane and the cell wall. As a result, in the modified cyanobacterium, protein and metabolites produced within the bacterial cell easily leak out to the outside of the bacterial cell because the outer membrane is easy to partially detach from the cell wall due to the weakened binding between the cell wall and the outer membrane. As a result, a plant acidic invertase activating substance produced within the bacterial cell also easily leaks out to the outside of the bacterial cell. Thus, the plant acidic invertase activator production method according to an aspect of the present disclosure can efficiently produce a plant acidic invertase activator because the modified cyanobacterium can easily secrete the plant acidic invertase activating substance.

For example, in the plant acidic invertase activator production method according to an aspect of the present disclosure, the gene encoding the SLH domain-containing outer membrane protein may be: slr1841 having a nucleotide sequence represented by SEQ ID NO: 7; nies970_09470 having a nucleotide sequence represented by SEQ ID NO: 8; anacy_3458 having a nucleotide sequence represented by SEQ ID NO: 9; or a gene having a nucleotide sequence that is at least 50 percent identical to the nucleotide sequence of any one of the slr1841, the nies970_09470, and the anacy_3458.

As a result, in the modified cyanobacterium, the gene encoding the SLH domain-containing outer membrane protein, represented by any one of SEQ ID NOs: 7 to 9 or a gene having a nucleotide sequence that is at least 50 percent identical to the nucleotide sequence of any one of these genes is deleted or inactivated. Hence, in the modified cyanobacterium, (i) the expression of any one of the SLH domain-containing outer membrane proteins described above or a protein functionally equivalent to any one of these proteins is suppressed, or (i) the function of any one of the SLH domain-containing outer membrane proteins described above or a protein functionally equivalent to any one of these proteins is suppressed or lost. As a result, in the modified cyanobacterium, the binding level and binding force of a binding domain (e.g., the SLH domain) of the outer membrane that binds to the cell wall are reduced. This facilitates partially detaching the outer membrane from the cell wall. As a result, protein and metabolites produced within the bacterial cell easily leak out to the outside of the bacterial cell, so that a plant acidic invertase activating substance produced within the bacterial cell also easily leaks out to the outside of the bacterial cell. Thus, the plant acidic invertase activator production method according to an aspect of the present disclosure can efficiently produce a plant acidic invertase activator because the plant acidic invertase activating substance produced within the bacterial cell of the modified cyanobacterium easily leaks out to the outside of the bacterial cell.

For example, in the plant acidic invertase activator production method according to an aspect of the present disclosure, the gene encoding the cell wall-pyruvic acid modifying enzyme may be: slr0688 having a nucleotide sequence represented by SEQ ID NO: 10; synpcc7942_1529 having a nucleotide sequence represented by SEQ ID NO: 11; anacy_1623 having a nucleotide sequence represented by SEQ ID NO: 12; or a gene having a nucleotide sequence that is at least 50 percent identical to the nucleotide sequence of any one of the slr0688, the synpcc7942_1529, and the anacy_1623.

As a result, in the modified cyanobacterium, the gene encoding the cell wall-pyruvic acid modifying enzyme, represented by any one of SEQ ID NOs: 10 to 12 or a gene having a nucleotide sequence that is at least 50 percent identical to the nucleotide sequence of any one of these enzyme-encoding genes is deleted or inactivated. Hence, in the modified cyanobacterium, (i) the expression of any one of the cell wall-pyruvic acid modifying enzymes described above or a protein functionally equivalent to any one of these enzymes is suppressed, or (ii) the function of any one of the cell wall-pyruvic acid modifying enzymes described above or a protein functionally equivalent to any one of these enzymes is suppressed or lost. A covalently linked sugar chain on the surface of the cell wall is thereby less susceptible to modification with pyruvic acid, so that binding level and binding force of the sugar chain of the cell wall that binds to the SLH domain of the SLH domain-containing outer membrane protein in the outer membrane are reduced. As a result, in the modified cyanobacterium, a decreased amount of a sugar chain on cell wall that binds to the outer membrane is modified with pyruvic acid, so that binding force between the cell wall and the outer membrane is weakened. This facilitates partially detaching the outer membrane from the cell wall. As a result, protein and metabolites produced within the bacterial cell easily leak out to the outside of the bacterial cell, so that a plant acidic invertase activating substance produced within the bacterial cell also easily leaks out to the outside of the bacterial cell. Thus, the plant acidic invertase activator production method according to an aspect of the present disclosure can efficiently produce a plant acidic invertase activator because the plant acidic invertase activating produced within the bacterial cell of the modified cyanobacterium easily leaks out to the outside of the bacterial cell.

Furthermore, a plant acidic invertase activator according to an aspect of the present disclosure includes: a secretion of a modified cyanobacterium in which a function of a protein involved in binding between an outer membrane and a cell wall of cyanobacterium is suppressed or lost.

As a result, the binding (i.e., binding level and binding force) between the cell wall and the outer membrane is partially reduced in the modified cyanobacterium. This facilitates partially detaching the outer membrane from the cell wall. Hence, in the modified cyanobacterium, protein and metabolites produced within the bacterial cell (i.e., intra-bacterial cell produced substances) easily leak out to the outside of the outer membrane (i.e., the outside of the bacterial cell). This facilitates secreting protein and metabolites produced within the bacterial cell of the modified cyanobacterium to the outside of the bacterial cell and therefore eliminates the need of extraction treatment of the intra-bacterial cell produced substances, such as the disruption of the bacterial cell. Hence, a plant acidic invertase activator containing a secretion of the modified cyanobacterium can be produced conveniently and efficiently. The intra-bacterial cell produced substances are less susceptible to reduction in physiological activity and yield because the extraction treatment of the intra-bacterial cell produced substances is unnecessary. Hence, a substance involved in activating acidic invertase of a plant (hereinafter, also referred to as a plant acidic invertase activating substance) among the intra-bacterial cell produced substances of the modified cyanobacterium is also less susceptible to reduction in physiological activity and yield. As a result, a plant acidic invertase activator having an improved plant acidic invertase activating effect can be obtained. Thus, the plant acidic invertase activator according to an aspect of the present disclosure can effectively activate acidic invertase of a plant.

Furthermore, a plant acidic invertase activation method according to an aspect of the present disclosure includes: using the above-described plant acidic invertase activator on a plant.

The plant acidic invertase activation method according to an aspect of the present disclosure uses a plant acidic invertase activator having an improved plant acidic invertase activating effect on a plant, and thus is capable of effectively activating the acidic invertase of plants.

Hereinafter, embodiments will be described in detail with reference to the drawings.

It should be noted that each of the subsequently described embodiments shows a generic or specific example. Numerical values, materials, steps, the processing order of the steps indicated in the following embodiments are merely examples, and thus are not intended to limit the present disclosure. Furthermore, among the elements in the following embodiments, elements that are not described in the independent claims indicating the broadest concepts are described as optional elements.

Furthermore, the figures are not necessarily precise illustrations. In the figures, elements that are substantially the same are given the same reference signs, and overlapping description thereof may be omitted or simplified.

Moreover, in the following embodiments, numerical ranges include, not only the precise meanings, but also substantially equal ranges, such as, for example, a measured amount (for example, the number, the concentration, etc.) a protein or a range thereof, etc.

In the present specification, both of a bacterial cell and a cell refer to one individual of cyanobacterium.

### [Embodiment]

In the present specification, the identity of a nucleotide sequence or an amino acid sequence is calculated with Basic Local Alignment Search Tool (BLAST) algorithm. Specifically, the identity is calculated by pairwise analysis with the BLAST program available in the website of the National Center for Biotechnology Information (NCBI) (https://blast.ncbi.nlm.nih.gov/Blast.cgi). Information on cyanobacterium genes and proteins encoded by these genes are published in, for example, the NCBI database mentioned above and Cyanobase (http://genome.microbedb.jp/cyanobase/). The amino acid sequence of the protein of interest and the nucleotide sequence of a gene encoding the protein can be obtained from these databases.

### [1. Plant acidic invertase activator]

First, the plant acidic invertase activator according to the present embodiment will be described. The plant acidic invertase activator contains a secretion involved in activating the acidic invertase of a plant, and has a plant acidic invertase activating effect. As mentioned above, invertase is an enzyme that catabolizes sucrose into reducing sugar such as glucose and fructose in plants. Particularly, acidic invertase contributes to the utilization of sucrose and catabolism into reducing sugar, a form of storage sugar, in plants. Thus, the plant acidic invertase activator according to the present embodiment activates the acidic invertase of a plant and is thereby capable of promoting plant growth and promoting storage sugar accumulation in fruits and the like. Hence, the plant acidic invertase activator according to the present embodiment is used in, for example, agricultural crops, and can thereby efficiently enhance the production of the agricultural crops.

For example, the promoting of plant growth refers to increasing the number of leaves, stems, buds, flowers, or fruits of a plant, thickening a stem or a trunk, and lengthening a height. The promoting of plant growth causes weight gain of a plant and its fruits and roots and increases the number of fruits.

Furthermore, acidic invertase contributes to the enhancement of plant quality such as plant disease control, enhancement of nutrient absorption, and higher sugar contents in fruits. Thus, the plant acidic invertase activator can effectively enhance plant quality such as increase in crop yield, weight gain of crops and fruits, higher sugar contents in fruits, reduction in physiological disorder, and reduction in disease for a plurality of crop species.

The plant includes crops that are cultivated in fields (so-called agricultural crops) as well as garden trees, flowers and ornamental plants, lawn, roadside trees, and the like, and also includes mountain forest trees which are rarely fertilized.

In the present embodiment, the plant acidic invertase activator comprises a secretion of a modified cyanobacterium in which a function of a protein involved in binding between an outer membrane and a cell wall of cyanobacterium (hereinafter, also referred to as parent cyanobacterium) is suppressed or lost. The cyanobacterium (i.e., parent cyanobacterium) and the modified cyanobacterium will be mentioned later.

As mentioned above, the secretion includes a secretion involved in activating acidic invertase of a plant. The secretion contains protein and metabolites produced within the bacterial cell of the modified cyanobacterium (i.e., intra-bacterial cell produced substances). The intra-bacterial cell produced substances include a substance involved in activating acidic invertase of a plant (i.e., a plant acidic invertase activating substance).

The plant acidic invertase activating substance is, for example, an organic substance degrading enzyme such as peptidase, nuclease, or phosphatase, a DNA metabolism-related substance such as adenosine or guanosine, an intracellular molecule involved in the promotion of nucleic acid (e.g., DNA or RNA) synthesis, such as p-aminobenzoic acid or spermidine, a ketone such as 3-hydroxybutyric acid, or an organic acid such as gluconic acid. The secretion of the modified cyanobacterium may be a mixture of these plant acidic invertase activating substances.

### [2. Plant acidic invertase activator production method]

Subsequently, the plant acidic invertase activator production method according to the present embodiment will be described with reference to FIG. 1. FIG. 1 is a flow chart illustrating one example of the plant acidic invertase activator production method according to the present embodiment.

The plant acidic invertase activator production method according to the present embodiment includes: preparing a modified cyanobacterium in which a function of a protein involved in binding between an outer membrane and a cell wall of cyanobacterium (i.e., parent cyanobacterium) is suppressed or lost (step S01); and causing the modified cyanobacteria to secrete a secretion involved in activating acidic invertase of a plant (step S02). As mentioned above, the secretion of the modified cyanobacterium contains protein and metabolites produced within the bacterial cell of the modified cyanobacterium (i.e., intra-bacterial cell produced substances). These intra-bacterial cell produced substances include a substance involved in activating acidic invertase of a plant (i.e., a plant acidic invertase activating substance).

In step S01, the modified cyanobacterium described above is prepared. The preparing of a modified cyanobacterium refers to adjusting the state of the modified cyanobacterium to a state where the modified cyanobacterium can secrete a secretion. The preparing of a modified cyanobacterium may be, for example, preparing the modified cyanobacterium by genetically modifying the parent cyanobacterium, may be reconstructing a bacterial cell from a freeze-dried form or a glycerol stock of the modified cyanobacterium, or may be retrieving the modified cyanobacterium that has finished secreting a plant acidic invertase activating substance in step S02.

In step S02, the modified cyanobacterium is caused to secrete a secretion involved in promoting growth of a plant. The modified cyanobacterium according to the present embodiment easily secretes protein and metabolites produced within the bacterial cell to the outside of the outer membrane (i.e., the outside of the bacterial cell) because a function of a protein involved in binding between an outer membrane and a cell wall of cyanobacterium (i.e., parent cyanobacterium) is suppressed or lost. These intra-bacterial cell produced substances also include a substance involved in activating acidic invertase of a plant. Hence, in step S02, the modified cyanobacterium is cultured under predetermined conditions and thereby caused to secrete intra-bacterial cell produced substances involved in activating acidic invertase of a plant to the outside of the bacterial cell.

Cyanobacterium culture can generally be carried out on the basis of liquid culture or a modified method thereof using a BG-11 medium (see Table 2). Hence, the culture of the modified cyanobacterium may be similarly carried out. The culture period of the cyanobacterium for plant acidic invertase activator production can be a period during which protein and metabolites accumulate with a high concentration under conditions where the bacterial cell has proliferated sufficiently, and may be, for example, 1 to 3 days or may be 4 to 7 days. A culture method may be, for example, aeration and agitation culture or shake culture.

The modified cyanobacterium thus cultured under the conditions described above produces protein and metabolites (i.e., intra-bacterial cell produced substances) within the bacterial cell and secretes the intra-bacterial cell produced substances into the culture solution. The intra-bacterial cell produced substances include an intra-bacterial cell produced substance involved in activating acidic invertase of a plant (i.e., a plant acidic invertase activating substance). In the case of retrieving the intra-bacterial cell produced substances secreted in the culture solution, insoluble materials such as the cell (i.e., the bacterial cell) may be removed from the culture solution by the filtration or centrifugation, etc. of the culture solution to retrieve a culture supernatant. The plant acidic invertase activator production method according to the present embodiment eliminates the need of disrupting the cell for plant acidic invertase activating substance retrieval because a secretion containing an intra-bacterial cell produced substance involved in activating acidic invertase of a plant (i.e., a plant acidic invertase activating substance) is secreted to the outside of the cell of the modified cyanobacterium. Hence, the modified cyanobacterium remaining after plant acidic invertase activating substance retrieval can be repeatedly used in plant acidic invertase activator production.

The method for retrieving the plant acidic invertase activating substance secreted into the culture solution is not limited to the example described above. While the modified cyanobacterium is cultured, the plant acidic invertase activating substance in the culture solution may be retrieved. For example, a protein-permeable membrane may be used to retrieve a plant acidic invertase activating substance that has passed through the permeable membrane. Thus, treatment to remove the bacterial cell of the modified cyanobacterium from a culture solution is unnecessary because, while the modified cyanobacterium is cultured, the plant acidic invertase activating substance in the culture solution can be retrieved. Hence, the plant acidic invertase activator can be produced more conveniently and efficiently.

Damage and stress on the modified cyanobacterium can be reduced because bacterial cell retrieval treatment from a culture solution and bacterial cell disruption treatment are unnecessary. Hence, the secretory productivity of a plant acidic invertase activating substance is less likely to be reduced in the modified cyanobacterium, and the modified cyanobacterium can be used for a longer time.

Thus, use of the modified cyanobacterium according to the present embodiment enables a plant acidic invertase activator to be obtained conveniently and efficiently.

Hereinafter, the cyanobacterium and the modified cyanobacterium will be described.

### [3. Cyanobacterium]

Cyanobacterium, also called blue-green alga or blue-green bacterium, is a group of prokaryote that collects light energy through chlorophyll and performs photosynthesis while generating oxygen through the splitting of water using the obtained energy. Cyanobacterium is highly diverse and includes, for example, unicellular species such as *Synechocystis* sp. PCC 6803 and filamentous species having multicellular filaments such as *Anabaena* sp. PCC 7120, in terms of cell shape. There are also thermophilic species such as *Thermosynechococcus elongatus,* marine species such as *Synechococcus elongatus,* and freshwater species such as *Synechocystis,* in terms of growth environment. Other examples thereof include many species having unique features, including species, such as *Microcystis aeruginosa,* which have a gas vesicle and produce toxin, and *Gloeobacter violaceus* which lacks thylakoid and has a light-harvesting antenna protein called phycobilisome in the plasma membrane.

FIG. 2 is a diagram schematically illustrating a cell surface of a cyanobacterium. As illustrated in FIG. 2, the cell surface of cyanobacterium is constituted by a plasma membrane (also referred to as inner membrane 1), peptidoglycan 2, and outer membrane 5 which is a lipid membrane that forms the outermost layer of the cell, in order from the inside. Sugar chain 3 constituted by glucosamine and mannosamine, etc. is covalently linked to peptidoglycan 2, and pyruvic acid is bound with this covalently linked sugar chain 3 (NPL 3: Jurgens and Weckesser, 1986, J. Bacteriol., 168: 568-573). In the present specification, peptidoglycan 2 and covalently linked sugar chain 3 are collectively referred to as cell wall 4. The space between the plasma membrane (i.e., inner membrane 1) and outer membrane 5 is called periplasm where various enzymes involved in protein degradation or conformation formation, lipid or nucleic acid degradation, or uptake of extracellular nutrients, etc. are present.

A SLH domain-containing outer membrane protein 6 (e.g., Slr1841 in the figure) has a C-terminal region embedded in a lipid membrane (also referred to as outer membrane 5) and N-terminal SLH domain 7 projecting from the lipid membrane, and is widely distributed in cyanobacterium and bacteria belonging to the class *Negativicutes,* a group of Gram-negative bacteria (NPL 4: Kojima et al., 2016, Biosci. Biotech. Biochem., 10: 1954-1959). The region embedded in the lipid membrane (i.e., outer membrane 5) forms a channel that allows hydrophilic materials to permeate the outer membrane, whereas SLH domain 7 has a function of binding to cell wall 4 (NPL 5: Kowata et al., 2017, J. Bacteriol., 199: e00371-17). The binding of SLH domain 7 to cell wall 4 requires modifying covalently linked sugar chain 3 on peptidoglycan 2 with pyruvic acid (NPL 6: Kojima et al., 2016, J. Biol. Chem., 291: 20198-20209). Examples of the gene encoding SLH domain-containing outer membrane protein 6 include slr1841 and slr1908 retained by *Synechocystis* sp. PCC 6803, and oprB retained by *Anabaena* sp. 90.

An enzyme that catalyzes the pyruvic acid modification reaction of covalently linked sugar chain 3 (hereinafter, referred to as cell wall-pyruvic acid modifying enzyme 9) in peptidoglycan 2 was identified in a Gram-positive bacterium *Bacillus anthracis* and designated as CsaB (NPL 7: Mesnage et al., 2000, EMBO J., 19: 4473-4484). Many species of cyanobacterium whose genomic nucleotide sequence is published retains a gene encoding a homologous protein having an amino acid sequence that has 30% or higher identity to the amino acid sequence of CsaB. Examples thereof include slr0688 retained by *Synechocystis* sp. PCC 6803 and syn7502_03092 retained by *Synechococcus* sp. 7502.

In cyanobacterium, CO₂ fixed by photosynthesis is converted to various amino acids and precursors of intracellular molecules through multiple stages of enzymatic reaction. Protein and metabolites are synthesized in the cytoplasm of cyanobacterium with these amino acids as starting materials. Such protein and metabolites include protein and metabolites that function in the cytoplasm and protein and metabolites that are transported from the cytoplasm to the periplasm and functions in the periplasm. However, any case where protein and metabolites are actively secreted to the outside of the cell has not been reported on cyanobacterium so far.

Cyanobacterium has high photosynthetic ability and therefore need not necessarily to take up organic substances as nutrients from the outside. Hence, cyanobacterium has only a very small amount of a channel protein, such as organic channel protein 8 (e.g., Slr1270) of FIG. 2, which permits permeation of organic substances, in outer membrane 5. For example, *Synechocystis* sp. PCC 6803 has only approximately 4% of organic channel protein 8 which permits permeation of organic substances based on the amount of total protein in outer membrane 5. On the other hand, outer membrane 5 of cyanobacterium is rich in an ion channel protein, such as SLH domain-containing outer membrane protein 6 (e.g., Slr1841) of FIG. 2, which permits selective permeation of only inorganic ions, for high-efficiency cellular uptake of inorganic ions necessary for growth. For example, in *Synechocystis* sp. PCC 6803, the ion channel protein which permits permeation of inorganic ions accounts for approximately 80% of the total protein of outer membrane 5.

Thus, cyanobacterium is considered to have the difficulty in actively secreting protein and metabolites produced within the bacterial cell to the outside of the bacterial cell, due to very few channels which permit permeation of organic substances such as protein in outer membrane 5.

### [4. Modified cyanobacterium]

Subsequently, the modified cyanobacterium according to the present embodiment will be described with reference to FIG. 2.

In the modified cyanobacterium according to the present embodiment, a function of a protein involved in binding between outer membrane 5 and cell wall 4 (hereinafter, also referred to as a binding-related protein) of cyanobacterium is suppressed or lost. As a result, the binding (e.g., binding level and binding force) between outer membrane 5 and cell wall 4 is partially reduced in the modified cyanobacterium. This facilitates partially detaching outer membrane 5 from cell wall 4. Hence, the modified cyanobacterium has improved secretory productivity of intra-bacterial cell produced substances to secrete protein and metabolites produced within the bacterial cell to the outside of the bacterial cell. As mentioned above, the intra-bacterial cell produced substances include an intra-bacterial cell produced substance involved in activating acidic invertase of a plant (i.e., a plant acidic invertase activating substance). Hence, the modified cyanobacterium also has improved secretory productivity of a plant acidic invertase activating substance that is produced within the bacterial cell and secreted to the outside of the bacterial cell. Furthermore, the modified cyanobacterium eliminates the need of retrieving a plant acidic invertase activating substance by disrupting the bacterial cell and can therefore be repeatedly used even after plant acidic invertase activating substance retrieval. In the present specification, to make protein and metabolites within the bacterial cell by the modified cyanobacterium is referred to as production, and to secrete the produced protein and metabolites to the outside of the bacterial cell is referred to as secretory production.

The protein involved in binding between outer membrane 5 and cell wall 4 may be at least one of SLH domain-containing outer membrane protein 6 or cell wall-pyruvic acid modifying enzyme 9. In the present embodiment, in the modified cyanobacterium, for example, the function of at least one of SLH domain-containing outer membrane protein 6 or cell wall-pyruvic acid modifying enzyme 9 is suppressed or lost. For example, in the modified cyanobacterium, (i) the function of at least one of SLH domain-containing outer membrane protein 6 or cell wall-pyruvic acid modifying enzyme 9 may be suppressed or lost, or (i) at least one of the expression of SLH domain-containing outer membrane protein 6 which binds to cell wall 4 or an enzyme that catalyzes the pyruvic acid modification reaction of a linked sugar chain on the surface of cell wall 4 (i.e., cell wall-pyruvic acid modifying enzyme 9) may be suppressed. As a result, the binding (e.g., binding level and binding force) between SLH domain 7 of SLH domain-containing outer membrane protein 6 in outer membrane 5 and covalently linked sugar chain 3 on the surface of cell wall 4 is reduced. Hence, outer membrane 5 is easily detached from cell wall 4 at a site having the weakened binding therebetween. Owing to partial detachment of outer membrane 5 from cell wall 4, intra-bacterial cell produced substances such as protein and metabolites present in the cell, particularly, the periplasm, of the modified cyanobacterium easily leaks out to the outside of the cell (outside of outer membrane 5). Accordingly, the modified cyanobacterium has improved secretory productivity of a plant acidic invertase activating substance that is produced within the bacterial cell and secreted to the outside of the bacterial cell.

Hereinafter, a cyanobacterium modified so as to partially detach outer membrane 5 from cell wall 4 by suppressing a function of at least one binding-related protein of SLH domain-containing outer membrane protein 6 and cell wall-pyruvic acid modifying enzyme 9 will be specifically described.

The type of the cyanobacterium before at least one of the expression of SLH domain-containing outer membrane protein 6 or the expression of cell wall-pyruvic acid modifying enzyme 9 is suppressed or lost (i.e., a parent cyanobacterium), which serves as the parent microbe of the modified cyanobacterium in the present embodiment, is not particularly limited and may be any type of cyanobacterium. The parent cyanobacterium may be, for example, the genus *Synechocystis, Synechococcus, Anabaena,* or *Thermosynechococcus,* and may be *Synechocystis* sp. PCC 6803, *Synechococcus* sp. PCC 7942, or *Thermosynechococcus elongatus* BP-1 among them.

The amino acid sequences of SLH domain-containing outer membrane protein 6 and the enzyme that catalyzes the pyruvic acid modification reaction of the cell wall (i.e., cell wall-pyruvic acid modifying enzyme 9) in the parent cyanobacterium, the nucleotide sequences of genes encoding these binding-related proteins, and the positions of the genes on chromosomal DNA or a plasmid can be confirmed in the NCBI database and Cyanobase mentioned above.

SLH domain-containing outer membrane protein 6 or cell wall-pyruvic acid modifying enzyme 9, the function of which is suppressed or lost in the modified cyanobacterium according to the present embodiment may be from any parent cyanobacterium and is not limited by the location where a gene encoding it resides (e.g., on chromosomal DNA or on a plasmid) as long as the parent cyanobacterium carries it.

SLH domain-containing outer membrane protein 6 may be, for example, Slr1841, Slr1908, or Slr0042 when the parent cyanobacterium is the genus *Synechocystis,* may be NIES970_09470, etc. when the parent cyanobacterium is the genus *Synechococcus,* may be Anacy_5815 or Anacy_3458, etc. when the parent cyanobacterium is the genus *Anabaena,* may be A0A0F6U6F8_MICAE, etc. when the parent cyanobacterium is the genus *Microcystis,* may be A0A3B8XX12_9CYAN, etc. when the parent cyanobacterium is the genus *Cyanothece,* may be A0A1Q8ZE23_9CYAN, etc. when the parent cyanobacterium is the genus *Leptolyngbya,* includes A0A1Z4R6U0_9CYAN when the parent cyanobacterium is the genus *Calothrix,* may be A0A1C0VG86_9NOSO, etc. when the parent cyanobacterium is the genus *Nostoc,* may be B1WRN6_CROS5, etc. when the parent cyanobacterium is the genus *Crocosphaera,* and may be K9TAE4_9CYAN, etc. when the parent cyanobacterium is the genus *Pleurocapsa.*

More specifically, SLH domain-containing outer membrane protein 6 may be, for example, Slr1841 (SEQ ID NO: 1) of *Synechocystis* sp. PCC 6803, NIES970_09470 (SEQ ID NO: 2) of *Synechococcus* sp. NIES-970, or Anacy_3458 (SEQ ID NO: 3) of *Anabaena cylindrica* PCC 7122. Alternatively, a protein having an amino acid sequence that is at least 50 percent identical to the amino acid sequence of any one of these SLH domain-containing outer membrane proteins 6 may be used.

As a result, in the modified cyanobacterium, for example, (i) the function of SLH domain-containing outer membrane protein 6 represented by any one of SEQ ID NOs: 1 to 3 or a protein having an amino acid sequence that is at least 50 percent identical to the amino acid sequence of any one of these SLH domain-containing outer membrane proteins 6 may be suppressed or lost, or (ii) the expression of SLH domain-containing outer membrane protein 6 represented by any one of SEQ ID NOs: 1 to 3 or a protein having an amino acid sequence that is at least 50 percent identical to the amino acid sequence of any one of these SLH domain-containing outer membrane proteins 6 may be suppressed. Hence, in the modified cyanobacterium, (i) the function of SLH domain-containing outer membrane protein 6 or a protein functionally equivalent to SLH domain-containing outer membrane protein 6 in outer membrane 5 is suppressed or lost, or (ii) the expression level of SLH domain-containing outer membrane protein 6 or a protein functionally equivalent to SLH domain-containing outer membrane protein 6 in outer membrane 5 is decreased. As a result, in the modified cyanobacterium, the binding level and binding force with which a binding domain (e.g., SLH domain 7) for binding outer membrane 5 with cell wall 4 binds to cell wall 4 are reduced. This facilitates partially detaching outer membrane 5 from cell wall 4. As a result, in the modified cyanobacterium, intra-bacterial cell produced substances easily leak out to the outside of the bacterial cell, so that a plant acidic invertase activating substance produced within the bacterial cell also easily leaks out to the outside of the bacterial cell.

In general, a protein having an amino acid sequence that is at least 30 percent identical to the amino acid sequence of a protein has high conformational homology to the protein and therefore, is reportedly likely to be functionally equivalent to the protein. Hence, SLH domain-containing outer membrane protein 6, the function of which is suppressed or lost may be, for example, a protein or a polypeptide which has an amino acid sequence that has 40% or higher, preferably 50% or higher, more preferably 60% or higher, further preferably 70% or higher, still further preferably 80% or higher, even further preferably 90% or higher identity to the amino acid sequence of SLH domain-containing outer membrane protein 6 represented by any one of SEQ ID NOs: 1 to 3, and which has a function of binding to covalently linked sugar chain 3 of cell wall 4.

Cell wall-pyruvic acid modifying enzyme 9 may be, for example, Slr0688 when the parent cyanobacterium is the genus *Synechocystis,* may be Syn7502_03092 or Synpcc7942_1529, etc. when the parent cyanobacterium is the genus *Synechococcus,* may be ANA_C20348 or Anacy_1623, etc. when the parent cyanobacterium is the genus *Anabaena,* may be CsaB (NCBI accession ID: TRU80220), etc. when the parent cyanobacterium is the genus *Microcystis,* may be CsaB (NCBI accession ID: WP_107667006.1), etc. when the parent cyanobacterium is the genus *Cyanothece,* may be CsaB (NCBI accession ID: WP_026079530.1), etc. when the parent cyanobacterium is the genus *Spirulina,* may be CsaB (NCBI accession ID: WP_096658142.1), etc. when the parent cyanobacterium is the genus *Calothrix,* may be CsaB (NCBI accession ID: WP_099068528.1), etc. when the parent cyanobacterium is the genus *Nostoc,* may be CsaB (NCBI accession ID: WP_012361697.1), etc. when the parent cyanobacterium is the genus *Crocosphaera,* and may be CsaB (NCBI accession ID: WP_036798735), etc. when the parent cyanobacterium is the genus *Pleurocapsa.*

More specifically, cell wall-pyruvic acid modifying enzyme 9 may be, for example, Slr0688 (SEQ ID NO: 4) of *Synechocystis* sp. PCC 6803, Synpcc7942_1529 (SEQ ID NO: 5) of *Synechococcus* sp. PCC 7942, or Anacy_1623 (SEQ ID NO: 6) of *Anabaena cylindrica* PCC 7122. Alternatively, a protein having an amino acid sequence that is at least 50 percent identical to the amino acid sequence of any one of these cell wall-pyruvic acid modifying enzymes 9 may be used.

As a result, in the modified cyanobacterium, for example, (i) the function of cell wall-pyruvic acid modifying enzyme 9 represented by any one of SEQ ID NOs: 4 to 6 or a protein having an amino acid sequence that is at least 50 percent identical to the amino acid sequence of any one of these cell wall-pyruvic acid modifying enzymes 9 may be suppressed or lost, or (ii) the expression of cell wall-pyruvic acid modifying enzyme 9 represented by any one of SEQ ID NOs: 4 to 6 or a protein having an amino acid sequence that is at least 50 percent identical to the amino acid sequence of any one of these cell wall-pyruvic acid modifying enzymes 9 may be suppressed. Hence, in the modified cyanobacterium, (i) the function of cell wall-pyruvic acid modifying enzyme 9 or a protein functionally equivalent to the enzyme is suppressed or lost, or (ii) the expression level of cell wall-pyruvic acid modifying enzyme 9 or a protein functionally equivalent to the enzyme is decreased. Covalently linked sugar chain 3 on the surface of cell wall 4 is thereby less susceptible to modification with pyruvic acid, so that binding level and binding force of sugar chain 3 of cell wall 4 that binds to SLH domain 7 of SLH domain-containing outer membrane protein 6 in outer membrane 5 are reduced. Therefore, in the modified cyanobacterium according to the present embodiment, covalently linked sugar chain 3 on the surface of cell wall 4 is less susceptible to modification with pyruvic acid, so that binding force between cell wall 4 and outer membrane 5 is weakened. This facilitates partially detaching outer membrane 5 from cell wall 4. As a result, in the modified cyanobacterium, intra-bacterial cell produced substances easily leak out to the outside of the bacterial cell, so that a plant growth promoting substance produced within the bacterial cell also easily leaks out to the outside of the bacterial cell.

As mentioned above, a protein having an amino acid sequence that is at least 30 percent identical to the amino acid sequence of a protein is reportedly likely to be functionally equivalent to the protein. Hence, cell wall-pyruvic acid modifying enzyme 9, the function of which is suppressed or lost may be, for example, a protein or a polypeptide which has an amino acid sequence that has 40% or higher, preferably 50% or higher, more preferably 60% or higher, further preferably 70% or higher, still further preferably 80% or higher, even further preferably 90% or higher identity to the amino acid sequence of cell wall-pyruvic acid modifying enzyme 9 represented by any one of SEQ ID NOs: 4 to 6, and which has a function of catalyzing reaction to modify covalently linked sugar chain 3 on peptidoglycan 2 of cell wall 4 with pyruvic acid.

In the present specification, the phrase "a function of SLH domain-containing outer membrane protein 6 is suppressed or lost" means that the ability of the protein to bind to cell wall 4 is suppressed or lost, the transport of the protein to outer membrane 5 is suppressed or lost, or the ability of the protein to be embedded so as to function in outer membrane 5 is suppressed or lost.

The phrase "a function of cell wall-pyruvic acid modifying enzyme 9 is suppressed or lost" means that the function of modifying covalently linked sugar chain 3 of cell wall 4 with pyruvic acid by the protein is suppressed or lost.

An approach for suppressing or losing the functions of these proteins is not particularly limited as long as the approach is usually used for suppressing or losing protein functions. The approach may involve, for example, deleting or inactivating a gene encoding SLH domain-containing outer membrane protein 6 and a gene encoding cell wall-pyruvic acid modifying enzyme 9, inhibiting the transcription of these genes, inhibiting the translation of transcripts of these genes, or administrating inhibitors which specifically inhibit these proteins.

In the present embodiment, in the modified cyanobacterium, outer membrane 5 and cell wall 4 cause, in the modified cyanobacterium, the expression of the protein involved in the binding between cell wall 4 and outer membrane 5 is suppressed, or the function of the protein is suppressed or lost. Therefore, the binding (i.e., binding level and binding force) between cell wall 4 and outer membrane 5 is partially reduced. As a result, in the modified cyanobacterium, outer membrane 5 is easy to partially detach from cell wall 4, so that intra-bacterial cell produced substances such as protein and metabolites produced within the bacterial cell of the modified cyanobacterium easily leak out to the outside of outer membrane 5, i.e., the outside of the bacterial cell. Hence, the modified cyanobacterium has improved secretory productivity of a plant acidic invertase activating substance that is produced within the bacterial cell and secreted to the outside of the bacterial cell. This eliminates the need of extraction treatment of the intra-bacterial cell produced substances, such as the disruption of the bacterial cell. Therefore, the intra-bacterial cell produced substances are less susceptible to reduction in physiological activity and yield. Hence, a plant acidic invertase activating substance produced within the bacterial cell is also less susceptible to reduction in physiological activity and yield. Therefore, a plant acidic invertase activator having an improved plant acidic invertase activating effect can be produced. The plant acidic invertase activating substance can be produced by repeatedly using the modified cyanobacterium even after retrieval of the intra-bacterial cell produced substances because the extraction treatment of the intra-bacterial cell produced substances is unnecessary.

The gene which causes expression of the protein involved in binding between outer membrane 5 and cell wall 4 may be, for example, at least one of a gene encoding SLH domain-containing outer membrane protein 6 or a gene encoding cell wall-pyruvic acid modifying enzyme 9. In the modified cyanobacterium, at least one of the gene encoding SLH domain-containing outer membrane protein 6 or the gene encoding cell wall-pyruvic acid modifying enzyme 9 is deleted or inactivated. Hence, in the modified cyanobacterium, for example, (i) the expression of at least one of SLH domain-containing outer membrane protein 6 or cell wall-pyruvic acid modifying enzyme 9 is suppressed, or (ii) the function of at least one of SLH domain-containing outer membrane protein 6 or cell wall-pyruvic acid modifying enzyme 9 is suppressed or lost. Hence, the binding (i.e., binding level and binding force) between SLH domain 7 of SLH domain-containing outer membrane protein 6 in outer membrane 5 and covalently linked sugar chain 3 on the surface of cell wall 4 is reduced. As a result, outer membrane 5 is easily detached from cell wall 4 at a site having the weakened binding between outer membrane 5 and cell wall 4. As a result, in the modified cyanobacterium, protein produced within the bacterial cell easily leaks out to the outside of the bacterial cell because outer membrane 5 is easy to partially detach from cell wall 4 by the weakened binding between outer membrane 5 and cell wall 4. As a result, a plant acidic invertase activating substance produced within the bacterial cell of the modified cyanobacterium also easily leaks out to the outside of the bacterial cell.

In the present embodiment, for example, the transcription of at least one of the gene encoding SLH domain-containing outer membrane protein 6 or the gene encoding cell wall-pyruvic acid modifying enzyme 9 may be suppressed in order to suppress or lose the function of at least one of SLH domain-containing outer membrane protein 6 or cell wall-pyruvic acid modifying enzyme 9 in cyanobacterium.

The gene encoding SLH domain-containing outer membrane protein 6 may be, for example, slr1841, slr1908, or slr0042 when the parent cyanobacterium is the genus *Synechocystis,* may be nies970_09470, etc. in the case of the genus *Synechococcus,* may be anacy_5815 or anacy_3458, etc. when the parent cyanobacterium is the genus *Anabaena,* may be A0A0F6U6F8_MICAE, etc. when the parent cyanobacterium is the genus *Microcystis,* may be A0A3B8XX12_9CYAN, etc. when the parent cyanobacterium is the genus *Cyanothece,* may be A0A1Q8ZE23_9CYAN, etc. when the parent cyanobacterium is the genus *Leptolyngbya,* may be A0A1Z4R6U0_9CYAN, etc. when the parent cyanobacterium is the genus *Calothrix,* may be A0A1C0VG86_9NOSO, etc. when the parent cyanobacterium is the genus *Nostoc,* may be B1WRN6_CROS5, etc. when the parent cyanobacterium is the genus *Crocosphaera,* and may be K9TAE4_9CYAN, etc. when the parent cyanobacterium is the genus *Pleurocapsa.* The nucleotide sequences of these genes can be obtained from the NCBI database or Cyanobase mentioned above.

More specifically, the gene encoding SLH domain-containing outer membrane protein 6 may be slr1841 (SEQ ID NO: 7) of *Synechocystis* sp. PCC 6803, nies970_09470 (SEQ ID NO: 8) of *Synechococcus* sp. NIES-970, anacy_3458 (SEQ ID NO: 9) of *Anabaena cylindrica* PCC 7122, or a gene having an amino acid sequence that is at least 50 percent identical to the amino acid sequence of any one of these genes.

As a result, in the modified cyanobacterium, the gene encoding SLH domain-containing outer membrane protein 6, represented by any one of SEQ ID NOs: 7 to 9 or a gene having a nucleotide sequence that is at least 50 percent identical to the nucleotide sequence of any one of these genes is deleted or inactivated. Hence, in the modified cyanobacterium, (i) the expression of any one of SLH domain-containing outer membrane proteins 6 described above or a protein functionally equivalent to any one of these proteins is suppressed, or (ii) the function of any one of SLH domain-containing outer membrane proteins 6 described above or a protein functionally equivalent to any one of these proteins is suppressed or lost. As a result, in the modified cyanobacterium, the binding level and binding force of a cell wall 4 binding domain (e.g., SLH domain 7) of outer membrane 5 that binds to cell wall 4 are reduced. This facilitates partially detaching outer membrane 5 from cell wall 4. As a result, protein and metabolites produced within the bacterial cell easily leak out to the outside of the bacterial cell, so that a plant acidic invertase activating substance produced within the bacterial cell also easily leaks out to the outside of the bacterial cell.

As mentioned above, a protein having an amino acid sequence that is at least 30 percent identical to the amino acid sequence of a protein is reportedly likely to be functionally equivalent to the protein. Hence, a gene having a nucleotide sequence that is at least 30 percent identical to the nucleotide sequence of a gene encoding a protein is considered likely to cause expression of a protein functionally equivalent to the protein. Hence, the gene encoding SLH domain-containing outer membrane protein 6, the function of which is suppressed or lost may be, for example, a gene which has a nucleotide sequence that has 40% or higher, preferably 50% or higher, more preferably 60% or higher, further preferably 70% or higher, still further preferably 80% or higher, even further preferably 90% or higher identity to the nucleotide sequence of the gene encoding SLH domain-containing outer membrane protein 6 represented by any one of SEQ ID NOs: 7 to 9, and which encodes a protein or a polypeptide having a function of binding to covalently linked sugar chain 3 of cell wall 4.

The gene encoding cell wall-pyruvic acid modifying enzyme 9 may be, for example, slr0688 when the parent cyanobacterium is the genus *Synechocystis,* may be syn7502_03092 or synpcc7942_1529, etc. when the parent cyanobacterium is the genus *Synechococcus,* may be ana_C20348 or anacy_1623, etc. when the parent cyanobacterium is the genus *Anabaena,* may be csaB (NCBI accession ID: TRU80220), etc. when the parent cyanobacterium is the genus *Microcystis,* may be csaB (NCBI accession ID: WP_107667006.1), etc. when the parent cyanobacterium is the genus *Cyanothece,* may be csaB (NCBI accession ID:WP_026079530.1), etc. when the parent cyanobacterium is the genus *Spirulina,* may be csaB (NCBI accession ID:WP_096658142.1), etc. when the parent cyanobacterium is the genus *Calothrix,* may be csaB (NCBI accession ID:WP_099068528.1), etc. when the parent cyanobacterium is the genus *Nostoc,* may be csaB (NCBI accession ID: WP_012361697.1), etc. when the parent cyanobacterium is the genus *Crocosphaera,* and may be csaB (NCBI accession ID: WP_036798735), etc. when the parent cyanobacterium is the genus *Pleurocapsa.* The nucleotide sequences of these genes can be obtained from the NCBI database or Cyanobase mentioned above.

More specifically, the gene encoding cell wall-pyruvic acid modifying enzyme 9 may be slr0688 (SEQ ID NO: 10) of *Synechocystis* sp. PCC 6803, synpcc7942_1529 (SEQ ID NO: 11) of *Synechococcus* sp. PCC 7942, or anacy_1623 (SEQ ID NO: 12) of *Anabaena cylindrica* PCC 7122. Alternatively, a gene having a nucleotide sequence that is at least 50 percent identical to the nucleotide sequence of any one of these genes may be used.

As a result, in the modified cyanobacterium, the gene encoding cell wall-pyruvic acid modifying enzyme 9, represented by any one of SEQ ID NOs: 10 to 12 or a gene having a nucleotide sequence that is at least 50 percent identical to the nucleotide sequence of any one of these enzyme-encoding genes is deleted or inactivated. Hence, in the modified cyanobacterium, (i) the expression of any one of cell wall-pyruvic acid modifying enzymes 9 described above or a protein functionally equivalent to any one of these enzymes is suppressed, or (ii) the function of any one of cell wall-pyruvic acid modifying enzymes 9 described above or a protein functionally equivalent to any one of these enzymes is suppressed or lost. Covalently linked sugar chain 3 on the surface of cell wall 4 is thereby less susceptible to modification with pyruvic acid, so that binding level and binding force of sugar chain 3 of cell wall 4 that binds to SLH domain 7 of SLH domain-containing outer membrane protein 6 in outer membrane 5 are reduced. Thus, in the modified cyanobacterium according to the present embodiment, a decreased amount of sugar chain 3 on cell wall 4 that binds to outer membrane 5 is modified with pyruvic acid, so that binding force between cell wall 4 and outer membrane 5 is weakened. This facilitates partially detaching outer membrane 5 from cell wall 4. As a result, protein and metabolites produced within the bacterial cell easily leak out to the outside of the bacterial cell, so that a plant acidic invertase activating substance produced within the bacterial cell also easily leaks out to the outside of the bacterial cell.

As mentioned above, a gene having a nucleotide sequence that is at least 30 percent identical to the nucleotide sequence of a gene encoding a protein is considered likely to cause expression of a protein functionally equivalent to the protein. Hence, the gene encoding cell wall-pyruvic acid modifying enzyme 9, the function of which is suppressed or lost may be, for example, a gene which has a nucleotide sequence that has 40% or higher, preferably 50% or higher, more preferably 60% or higher, further preferably 70% or higher, still further preferably 80% or higher, even further preferably 90% or higher identity to the nucleotide sequence of the gene encoding cell wall-pyruvic acid modifying enzyme 9 represented by any one of SEQ ID NOs: 10 to 12, and which encodes a protein or a polypeptide having a function of catalyzing reaction to modify covalently linked sugar chain 3 on peptidoglycan 2 of cell wall 4 with pyruvic acid.

### [5. Modified cyanobacterium production method]

Subsequently, the modified cyanobacterium production method according to the present embodiment will be described. The modified cyanobacterium production method includes causing a function of a protein involved in binding between outer membrane 5 and cell wall 4 of cyanobacterium to be suppressed or lost.

In the present embodiment, in the (i), the protein involved in binding between outer membrane 5 and cell wall 4 may be, for example, at least one of SLH domain-containing outer membrane protein 6 or cell wall-pyruvic acid modifying enzyme 9.

An approach for suppressing or losing the function of the protein is not particularly limited and may involve, for example, deleting or inactivating a gene encoding SLH domain-containing outer membrane protein 6 and a gene encoding cell wall-pyruvic acid modifying enzyme 9, inhibiting the transcription of these genes, inhibiting the translation of transcripts of these genes, or administrating inhibitors which specifically inhibit these proteins.

An approach for deleting or inactivating the gene may be, for example, the mutagenesis of one or more bases on the nucleotide sequence of the gene, the substitution of the nucleotide sequence by another nucleotide sequence, the insertion of another nucleotide sequence thereto, or the partial or complete deletion of the nucleotide sequence of the gene.

An approach for inhibiting the transcription of the gene may be, for example, the mutagenesis of a promoter region of the gene, the inactivation of the promoter by substitution by another nucleotide sequence or insertion of another nucleotide sequence, or CRISPR interference (NPL 8: Yao et al., ACS Synth. Biol., 2016, 5: 207-212). A specific approach for the mutagenesis or the substitution by or insertion of a nucleotide sequence may be, for example, ultraviolet irradiation, site-directed mutagenesis, or homologous recombination.

An approach for inhibiting the translation of a transcript of the gene may be, for example, RNA (ribonucleic acid) interference.

The function of the protein involved in binding between outer membrane 5 and cell wall 4 of cyanobacterium may be suppressed or lost to produce the modified cyanobacterium by use of any one of the above approaches.

As a result, the binding (e.g., binding level and binding force) between cell wall 4 and outer membrane 5 is partially reduced in the modified cyanobacterium produced by the production method described above. This facilitates partially detaching outer membrane 5 from cell wall 4. As a result, in the modified cyanobacterium, intra-bacterial cell produced substances such as protein and metabolites produced within the bacterial cell easily leak out to the outside of outer membrane 5 (i.e., the outside of the bacterial cell), so that a substance involved in activating acidic invertase of a plant (i.e., a plant acidic invertase activating substance) also easily leaks out to the outside of the bacterial cell. Thus, the modified cyanobacterium production method according to the present embodiment can provide a modified cyanobacterium having improved secretory productivity of a plant acidic invertase activating substance.

The modified cyanobacterium produced by the production method in the present embodiment eliminates the need of disrupting the bacterial cell for plant acidic invertase activating substance retrieval because plant acidic invertase activating substance produced within the bacterial cell easily leaks out to the outside of the bacterial cell. For example, the modified cyanobacterium can be cultured under appropriate conditions, and subsequently, plant acidic invertase activating substance secreted into the culture solution can be retrieved. Therefore, while the modified cyanobacterium is cultured, the plant acidic invertase activating substance in the culture solution may be retrieved. Hence, use of the modified cyanobacterium obtained by this production method enables efficient microbiological plant acidic invertase activating substance production to be carried out. Thus, the modified cyanobacterium production method in the present embodiment can provide a modified cyanobacterium with high use efficiency that can be repeatedly used even after plant acidic invertase activating substance retrieval.

### [6. Plant acidic invertase activation method]

The plant acidic invertase activation method according to the present embodiment includes using the plant acidic invertase activator described above on a plant. As mentioned above, use of the plant acidic invertase activator on a plant can effectively cause activation of plant acidic invertase because the plant acidic invertase activator according to the present embodiment is a plant acidic invertase activator having an improved plant acidic invertase activating effect.

The plant acidic invertase activator described above may be used as it is, as a matter of course, or may be used after being concentrated or diluted. For the use (application) of the plant acidic invertase activator to a plant, the concentration and application method of the plant acidic invertase activator may be appropriately determined according to the type of the plant, the properties of soil, and purpose, etc. The plant acidic invertase activator may be, for example, a culture solution itself of the modified cyanobacterium, may be a solution obtained by removing the bacterial cell of the modified cyanobacterium from the culture solution, or may be extracts obtained by extracting a desired substance from the culture solution by a membrane technique or the like. The desired substance may be an enzyme that degrades nutrients in soil, may be a substance (e.g., a substance having a chelating effect) that solubilizes an insoluble substance (e.g., a metal such as iron) in soil, or may be a substance that improves the intracellular physiological activity of a plant. The method for applying the plant acidic invertase activator to a plant may be, for example, spraying onto the plant, or spraying, irrigation, or mixing to soil, or mixing into a hydroponic solution. For example, for each plant individual, several mL of the plant acidic invertase activator may be added to the base of the plant approximately once a week.

### [Working examples]

Hereinafter, the modified cyanobacterium, the modified cyanobacterium production method, and the plant acidic invertase activator production method of the present disclosure will be specifically described with reference to working examples. However, the present disclosure is not limited by the following working examples by any means.

In the following working examples, two types of modified cyanobacteria were produced by suppressing the expression of slr1841 gene encoding a SLH domain-containing outer membrane protein (Example 1) and suppressing the expression of slr0688 gene encoding a cell wall-pyruvic acid modifying enzyme (Example 2) as methods for partially detaching the outer membrane of cyanobacterium from the cell wall. Then, the measurement of secretory productivity of protein and the identification of the secreted intra-bacterial cell produced substances (here, protein and intracellular metabolites) were performed as to these modified cyanobacteria. The cyanobacterium species used in the present working examples is *Synechocystis* sp. PCC 6803 (hereinafter, simply referred to as "cyanobacterium").

### (Example 1)

In Example 1, a modified cyanobacterium was produced in which the expression of slr1841 gene encoding a SLH domain-containing outer membrane protein was suppressed.

### (1) Construction of modified cyanobacterium strain in which expression of slr1841 gene was suppressed

The gene expression suppression method used was CRISPR (Clustered Regularly Interspaced Short Palindromic Repeat) interference. In this method, the expression of the slr1841 gene can be suppressed by introducing a gene encoding dCas9 protein (hereinafter, referred to as dCas9 gene) and slr1841_sgRNA (single-guide ribonucleic acid) gene to the chromosomal DNA of cyanobacterium.

The mechanism of the suppression of gene expression by this method is as follows.

First, nuclease activity-deficient Cas9 protein (dCas9) and sgRNA (slr1841_sgRNA) complementarily binding to the nucleotide sequence of the slr1841 gene forms a complex.

Next, this complex recognizes the slr1841 gene on the chromosomal DNA of cyanobacterium and specifically binds to the slr1841 gene. This binding, which serves as steric hindrance, inhibits the transcription of the slr1841 gene. As a result, the expression of the slr1841 gene in the cyanobacterium is suppressed.

Hereinafter, a method for introducing each of the two genes described above to the chromosomal DNA of cyanobacterium will be specifically described.

### (1-1) Introduction of dCas9 gene

The dCas9 gene, operator gene for the expression control of the dCas9 gene, and spectinomycin resistance marker gene serving as an indicator for gene introduction were amplified by PCR (polymerase chain reaction) with the chromosomal DNA of a *Synechocystis* LY07 strain (hereinafter, also referred to as an LY07 strain) (see NPL 8) as a template using the primers psbA1-Fw (SEQ ID NO: 13) and psbA1-Rv (SEQ ID NO: 14) described in Table 1. In the LY07 strain, these three genes are inserted in a linked state in psbA1 gene on the chromosomal DNA and can therefore be amplified as one DNA fragment by PCR. Here, the obtained DNA fragment is referred to as a "psbA1::dCas9 cassette". The psbA1::dCas9 cassette was inserted to a pUC19 plasmid by use of In-Fusion PCR Cloning(R) to obtain a pUC19-dCas9 plasmid.

**[Table 1]**

| **Primer name** | **Nucleotide sequence** | **SEQ ID NO** |
|---|---|---|
| psbA1-Fw | 5' -CAGTGAATTCGAGCTCGGTATATAGCGTTGCAGTCCCTGG-3' | 13 |
| psbA1-Rv | 5' -GATTACGCCAAGCTTGCATGACCGCGGTCACTTCATAACC-3' | 14 |
| slr2030-Fw | 5' -CAGTGAATTCGAGCTCGGTAATAACCGTTGTCCCTTTTGTTTCATCG-3' | 15 |
| sgRNA_slr1841-Rv | 5' -TGTTAGTGAGCCCTGCTGTTAGCTCCCAGTATCTCTATCACTGAT-3' | 16 |
| sgRNA_slr1841-Fw | 5' -ACAGCAGGGCTCACTAACAGTTTTAGAGCTAGAAATAGCAAGTTAAAATAA-3' | 17 |
| slr2031-Rv | 5' -GATTACGCCAAGCTTGCATGGGGAACAAGCTGAATCTGGGCATC-3' | 18 |
| sgRNA₋slr0688-Rv | 5' -TTTTAGTCTGTTTGCTGGATAGCTCCCAGTATCTCTATCACTGAT-3' | 19 |
| sgRNA_slr0688-Fw | 5' -TGCAGCAAACAGACTAAAAGTTTTAGAGCTAGAAATAGCAAGTTAAAATAA-3' | 20 |

1 µg of the obtained pUC19-dCas9 plasmid and a cyanobacterium culture solution (bacterial cell concentration OD730 = approximately 0.5) were mixed, and the pUC19-dCas9 plasmid was introduced into the cyanobacterium cells by spontaneous transformation. The transformed cells were selected by growth on a BG-11 agar medium containing 20 µg/mL spectinomycin. In the selected cells, homologous recombination occurred between the psbA1 gene on the chromosomal DNA and the upstream and downstream fragment regions of psbA1 on the pUC19-dCas9 plasmid. In this way, a *Synechocystis* dCas9 strain having the insert of the dCas9 cassette in the psbA1 gene region was obtained. The composition of the BG-11 medium used is as described in Table 2.

**[Table 2]**

| **Component** | **Content (mg/L)** |
|---|---|
| EDTA-Na | 1 |
| Ammonium Iron Citrate | 6 |
| NaNO₃ | 1500 |
| MgSO₄ | 75 |
| K₂HPO₄ | 39 |
| CaCl₂ | 28.6 |
| H₃BO₄ | 2.8 |
| MnCl₂ | 1.8 |
| ZnSO₄ | 0.2 |
| CuSO₄ | 0.08 |
| Na₂MoO₄ | 0.02 |
| Co(NO₃)₂ | 0. 005 |
| TES-KOH (pH 7.5) | 4580 |

### (1-2) Introduction of slr1841_sgRNA gene

In the CRISPR interference, sgRNA specifically binds to a target gene by introducing a sequence of approximately 20 bases complementary to the target sequence to a region called protospacer on the sgRNA gene. The protospacer sequence used in the present working examples is described in Table 3.

**[Table 3]**

| **Protospacer target gene** | **Nucleotide sequence** | **SEQ ID NO** |
|---|---|---|
| slr1841 | 5' -ACAGCAGGGCTCACTAACA-3' | 21 |
| slr0688 | 5' -TGCAGCAAACAGACTAAAA-3' | 22 |

In the *Synechocystis* LY07 strain, the sgRNA gene (except for the protospacer region) and kanamycin resistance marker gene are inserted in a linked state in slr2030-slr2031 genes on the chromosomal DNA. Thus, sgRNA that specifically recognizes slr1841 (slr1841_sgRNA) can be easily obtained by adding a protospacer sequence (SEQ ID NO: 21) complementary to the slr1841 gene (SEQ ID NO: 7) to primers for use in amplifying the sgRNA gene by PCR.

First, two DNA fragments were amplified by PCR with the chromosomal DNA of the LY07 strain as a template using a set of the primers slr2030-Fw (SEQ ID NO: 15) and sgRNA_slr1841-Rv (SEQ ID NO: 16) and a set of the primers sgRNA_slr1841-Fw (SEQ ID NO: 17) and slr2031-Rv (SEQ ID NO: 18) descried in Table 1.

Subsequently, a DNA fragment (slr2030-2031::slr1841_sgRNA) having (i) the slr2030 gene fragment, (ii) slr1841_sgRNA, (iii) kanamycin resistance marker gene, and (iv) the slr2031 gene fragment linked in order was obtained by PCR amplification with a mixed solution of the DNA fragments described above as a template using the primers slr2030-Fw (SEQ ID NO: 15) and slr2031-Rv (SEQ ID NO: 18) described in Table 1. The slr2030-2031::s1r1841_sgRNA was inserted to a pUC19 plasmid by use of In-Fusion PCR Cloning(R) to obtain a pUC19- slr1841_sgRNA plasmid.

The pUC19-slr1841_sgRNA plasmid was introduced to the *Synechocystis* dCas9 strain in the same manner as in the (1-1), and the transformed cells were selected on a BG-11 agar medium containing 30 µg/mL kanamycin. In this way, a transformant *Synechocystis* dCas9 slr1841_sgRNA strain having the insert of slr1841_sgRNA in the slr2030-slr2031 gene on the chromosomal DNA (hereinafter, also referred to as a slr1841-suppressed strain) was obtained.

### (1-3) Suppression of slr1841 gene

The promoter sequences of the dCas9 gene and the slr1841_sgRNA gene were designed such that expression was induced in the presence of anhydrotetracycline (aTc). In the present working examples, the expression of the slr1841 gene was suppressed by adding aTc (final concentration: 1 µg/mL) into the medium.

### (Example 2)

In Example 2, a modified cyanobacterium in which the expression of slr0688 gene encoding a cell wall-pyruvic acid modifying enzyme was suppressed was obtained by the following procedures.

### (2) Construction of modified cyanobacterium strain in which expression of slr0688 gene was suppressed

sgRNA gene containing a protospacer sequence (SEQ ID NO: 22) complementary to the slr0688 gene (SEQ ID NO: 4) was introduced to the *Synechocystis* dCas9 strain by the same procedures as in the (1-2) to obtain a *Synechocystis* dCas9 slr0688_sgRNA strain. The same conditions as in the (1-2) were used except that a set of the primers slr2030-Fw (SEQ ID NO: 15) and sgRNA_slr0688-Rv (SEQ ID NO: 19) and a set of the primers sgRNA_slr0688-Fw (SEQ ID NO: 20) and slr2031-Rv (SEQ ID NO: 18) described in Table 1 were used; and a DNA fragment (slr2030-2031::slr0688_sgRNA) having (i) the slr2030 gene fragment, (ii) slr0688_sgRNA, (iii) kanamycin resistance marker gene, and (iv) the slr2031 gene fragment linked in order was inserted to a pUC19 plasmid by use of In-Fusion PCR Cloning(R) to obtain a pUC19-slr0688_sgRNA plasmid.

Further, the expression of the slr0688 gene was suppressed by the same procedures as in the (1-3).

### (Comparative example 1)

In Comparative Example 1, the *Synechocystis* dCas9 strain was obtained by the same procedures as in (1-1) of Example 1.

Subsequently, the state of cell surface was observed and a protein secretory productivity test was performed as to each of the bacterial strains obtained in Example 1, Example 2 and Comparative Example 1. Hereinafter, the details will be described.

### (3) Observation of state of cell surface of bacterial strain

The respective ultrathin sections of the modified cyanobacterium *Synechocystis* dCas9 slr1841_sgRNA strain (i.e., a slr1841-suppressed strain) obtained in Example 1, the modified cyanobacterium *Synechocystis* dCas9 slr0688_sgRNA strain (hereinafter, also referred to as a slr0688-suppressed strain) obtained in Example 2, and the modified cyanobacterium *Synechocystis* dCas9 strain (hereinafter, referred to as a control strain) obtained in Comparative Example 1 were prepared, and the state of cell surface (in other words, an outer membrane structure) was observed under an electron microscope.

### (3-1) Culture of bacterial strain

The slr1841-suppressed strain of Example 1 was inoculated at initial bacterial cell concentration OD730 = 0.05 to a BG-11 medium containing 1 µg/mL aTc and shake-cultured for 5 days under conditions of a light quantity of 100 µmol/m2/s and 30°C. The slr0688-suppressed strain of Example 2 and the control strain of Comparative Example 1 were also cultured under the same conditions as in Example 1.

### (3-2) Preparation of ultrathin section of bacterial strain

The culture solution obtained in the (3-1) was centrifuged at 2,500 g at room temperature for 10 minutes to retrieve the cells of the slr1841-suppressed strain of Example 1. Subsequently, the cells were rapidly frozen with liquid propane of -175°C and then fixed at -80°C for 2 days using an ethanol solution containing 2% glutaraldehyde and 1% tannic acid. The cells thus fixed were dehydrated with ethanol, and the dehydrated cells were impregnated with propylene oxide and then immersed in a resin (Quetol-651) solution. Then, the resin was cured by still standing at 60°C for 48 hours to embed the cells in the resin. The cells in the resin were sliced into a thickness of 70 nm using an ultramicrotome (Ultracut) to produce an ultrathin section. This ultrathin section was stained using 2% uranium acetate and 1% lead citrate solutions to provide a transmission electron microscopy sample of the slr1841-suppressed strain of Example 1. The slr0688-suppressed strain of Example 2 and the control strain of Comparative Example 1 were also each subjected to the same operation as above to provide transmission electron microscopy samples.

### (3-3) Observation under electron microscope

The ultrathin sections obtained in the (3-2) were observed under an accelerating voltage of 100 kV using a transmission electron microscope (JEOL JEM-1400Plus). The observation results are shown in FIGs. 3 to 8.

First, the slr1841-suppressed strain of Example 1 will be described. FIG. 3 is a TEM (transmission electron microscope) image of the slr1841-suppressed strain of Example 1. FIG. 4 is an enlarged image of broken line region A of FIG. 3. (a) in FIG. 4 is an enlarged TEM image of broken line region A of FIG. 3, and (b) in FIG. 4 is a diagram graphically depicting the enlarged TEM image of (a) in FIG. 4.

As illustrated in FIG. 3, in the slr1841-suppressed strain of Example 1, the outer membrane was partially stripped (i.e., the outer membrane partially came off) from the cell wall while the outer membrane became partially loose.

In order to confirm the more detailed state of cell surface, broken line region A was subjected to magnifying observation. As a result, as illustrated in (a) and (b) of FIG. 4, sites where the outer membrane partially came off (dot-dash line regions a1 and a2 in the figures) were able to be confirmed. Also, a site where the outer membrane became largely loose was able to be confirmed near dot-dash line region a1. This site is a site having weakened binding between the outer membrane and the cell wall. It is considered that the outer membrane swelled outward and became loose because the culture solution infiltrated into the periplasm from the outer membrane.

Subsequently, the slr0688-suppressed strain of Example 2 will be described. FIG. 5 is a TEM image of the slr0688-suppressed strain of Example 2. FIG. 6 is an enlarged image of broken line region B of FIG. 5. (a) in FIG. 6 is an enlarged TEM image of broken line region B of FIG. 5, and (b) in FIG. 6 is a diagram graphically depicting the enlarged TEM image of (a) in FIG. 6.

As illustrated in FIG. 5, in the slr0688-suppressed strain of Example 2, the outer membrane was partially stripped from the cell wall while the outer membrane partially became loose. In the slr0688-suppressed strain, it was able to be confirmed that the outer membrane was partially detached from the cell wall.

In order to confirm the more detailed state of cell surface, broken line region B was subjected to magnifying observation. As a result, as illustrated in (a) and (b) in FIG. 6, a site where the outer membrane became largely loose (dot-dash line region b1 in the figures) and sites where the outer membrane partially came off (dot-dash line regions b2 and b3 in the figures) were able to be confirmed. Also, a site where the outer membrane was detached from the cell wall was able to be confirmed near each of dot-dash line regions b1, b2, and b3.

Subsequently, the control strain of Comparative Example 1 will be described. FIG. 7 is a TEM image of the control strain of Comparative Example 1. FIG. 8 is an enlarged image of broken line region C of FIG. 7. (a) in FIG. 8 is an enlarged TEM image of broken line region C of FIG. 7, and (b) in FIG. 8 is a diagram graphically depicting the enlarged TEM image of (a) in FIG. 8.

As illustrated in FIG. 7, the control strain of Comparative Example 1 had ordered cell surface where the inner membrane, the cell wall, the outer membrane, and the S-layer were kept in a state layered in order. Specifically, the control strain exhibited none of the site where the outer membrane was detached from the cell wall, the site where the outer membrane was stripped (i.e., came off) from the cell wall, and the site where the outer membrane became loose, which were found in Examples 1 and 2.

### (4) Protein secretory productivity test

The slr1841-suppressed strain of Example 1, the slr0688-suppressed strain of Example 2, and the control strain of Comparative Example 1 were each cultured, and the amount of protein secreted to the outside of the cells (hereinafter, also referred to as the amount of secretory protein) was measured. Each of the bacterial strains described above was evaluated for the secretory productivity of protein from the amount of protein in the culture solution. The secretory productivity of protein refers to the ability to produce protein by secreting intracellularly produced protein to the outside of the cells. Hereinafter, a specific method will be described.

### (4-1) Culture of bacterial strain

The slr1841-suppressed strain of Example 1 was cultured in the same manner as in the (3-1). The culture was performed three independent times. The bacterial strains of Example 2 and Comparative Example 1 were also cultured under the same conditions as in the bacterial strain of Example 1.

### (4-2) Quantification of protein secreted to outside of cell

Each culture solution obtained in the (4-1) was centrifuged at 2,500 g at room temperature for 10 minutes to obtain a culture supernatant. The obtained culture supernatant was filtered through a membrane filter having a pore size of 0.22 µm to completely remove the cells of the slr1841-suppressed strain of Example 1. The amount of total protein contained in the culture supernatant thus filtered was quantified by the BCA (bicinchoninic acid) method. This series of operations was performed as to each of the three culture solutions obtained by culture performed three independent times to determine a mean and standard deviation of the amounts of protein secreted to the outside of the cells of the slr1841-suppressed strain of Example 1. The protein in the three culture solutions were also quantified under the same conditions as above as to each of the bacterial strains of Example 2 and Comparative Example 1, and a mean and standard deviation of the amounts of protein in the three culture solutions was determined.

The results are shown in FIG. 9. FIG. 9 is a graph illustrating the amount of protein (n = 3, error bar = SD) in the culture supernatant of a modified cyanobacterium in Example 1, Example 2, and Comparative Example 1.

As illustrated in FIG. 9, the amount (mg/L) of protein secreted into the culture supernatant was improved by approximately 25 times in all the slr1841-suppressed strain of Example 1 and the slr0688-suppressed strain of Example 2 compared with the control strain of Comparative Example 1.

Although the description of data is omitted, the absorbance (730 nm) of the culture solution was measured and the amount of secretory protein per g of bacterial cell dry weight (mg protein/g cell dry weight) was calculated. As a result, the amount of secretory protein per g of bacterial cell dry weight (mg protein/g cell dry weight) was improved by approximately 36 times in all the slr1841-suppressed strain of Example 1 and the slr0688-suppressed strain of Example 2 compared with the control strain of Comparative Example 1.

Furthermore, as illustrated in FIG. 9, the amount of protein secreted into the culture supernatant was larger for the slr0688-suppressed strain of Example 2 in which the expression of the gene encoding the cell wall-pyruvic acid modifying enzyme (slr0688) was suppressed than for the slr1841-suppressed strain of Example 1 in which the expression of the gene encoding the SLH domain-containing outer membrane protein (slr1841) was suppressed. This is probably related to a larger number of covalently linked sugar chains on cell wall surface than the number of the SLH domain-containing outer membrane protein (Slr1841) in the outer membrane. Specifically, the amount of protein secreted was increased from that for the slr1841-suppressed strain of Example 1 probably because the slr0688-suppressed strain of Example 2 had smaller binding level and binding force between the outer membrane and the cell wall than those of the slr1841-suppressed strain of Example 1.

From these results, it was able to be confirmed that a function of a protein related to binding between an outer membrane and a cell wall is suppressed, whereby the binding between the outer membrane and the cell wall of cyanobacterium is partially weakened so that the outer membrane is partially detached from the cell wall. It was also able to be confirmed that the weakened binding between the outer membrane and the cell wall facilitates leaking intracellularly produced protein of cyanobacterium to the outside of the cell. Thus, the modified cyanobacterium and the production method thereof according to the present embodiment were found to largely improve the secretory productivity of protein.

### (5) Identification of secreted protein

Subsequently, the secreted protein contained in the culture supernatant obtained in the (4-2) was identified by LC-MS/MS. A method will be described below.

### (5-1) Sample preparation

Cold acetone was added in an amount of 8 times the fluid volume of the culture supernatant, and the mixture was left standing at 20°C for 2 hours and then centrifuged at 20,000 g for 15 minutes to obtain a precipitate of the protein. To this precipitate, 100 mM Tris pH 8.5 and 0.5% sodium dodecanoate (SDoD) were added, and the protein was lysed with a closed sonicator. After adjustment of the protein concentration to 1 µg/mL, dithiothreitol (DTT) (final concentration: 10 mM) was added thereto, and the mixture was left standing at 50°C for 30 minutes. Subsequently, iodoacetamide (IAA) (final concentration: 30 mM) was added thereto, and the mixture was left standing at room temperature (in the dark) for 30 minutes. In order to terminate the reaction of IAA, cysteine (final concentration: 60 mM) was added thereto, and the mixture was left standing at room temperature for 10 minutes. 400 ng of trypsin was added thereto, and the mixture was left standing overnight at 37°C to convert the protein to peptide fragments. After addition of 5% TFA (trifluoroacetic acid), the mixture was centrifuged at 15,000 g at room temperature for 10 minutes to obtain a supernatant. By this operation, SDoD was removed. The sample was desalted using a C18 spin column and then dried with a centrifugal evaporator. Then, 3% acetonitrile and 0.1% formic acid were added thereto, and the sample was lysed using a closed sonicator. The peptide concentration was adjusted to 200 ng/µL.

### (5-2) LC-MS/MS analysis

The sample obtained in the (5-1) was analyzed using an LC-MS/MS apparatus (UltiMate 3000 RSLCnano LC System) under the following conditions.

Amount of sample injected: 200 ng
Column: CAPCELL CORE MP 75 µm × 250 mm
Solvent: solvent A: 0.1% aqueous formic acid solution, solvent B: 0.1% formic acid + 80% acetonitrile
Gradient program: 8% solvent B 4 minutes after sample injection, 44% solvent B 27 minutes later, 80% solvent B 28 minutes later, and completion of measurement 34 minutes later.

### (5-3) Data analysis

The obtained data was analyzed under the following conditions to perform protein and peptide identification and the calculation of quantification values.

Software: Scaffold DIA
Database: UniProtKB/Swiss Prot database (*Synechocystis* sp. PCC 6803)
Fragmentation: HCD
Precursor Tolerance: 8 ppm
Fragment Tolerance: 10 ppm
Data Acquisition Type: Overlapping DIA
Peptide Length: 8-70
Peptide Charge: 2-8
Max Missed Cleavages: 1
Fixed Modification: Carbamidomethylation
Peptide FDR: 1% or less

Among the identified proteins, proteins predicted to have evident enzymatic activity among 30 types of proteins having the largest relative quantification values are described in Table 4.

**[Table 4]**

| | **Protein name** | **Uniprot Accession ID** | **Gene name** |
|---|---|---|---|
| 1 | Carboxyl-terminal protease | P73458 | prc |
| 2 | Iron uptake prote i n A2 | 055835 | futA2 |
| 3 | Extracellular nuclease | P72938 | nucH |
| 4 | Alkaline phosphatase | P72939 | sII0654 |
| 5 | N-acetylmuramoyl-L-alanine amidase | P73736 | amiA |
| 6 | Peptidyl-prolyl cis-trans isomerase | P73037 | ytfC |

All these 6 types of proteins were contained in each of the culture supernatants of the slr1841-suppressed strain of Example 1 and the slr0688-suppressed strain of Example 2. All of these proteins retained a periplasm (which refers to the space between the outer membrane and the inner membrane) localization signal. From these results, it was able to be confirmed that in the modified strains of Example 1 and Example 2, the outer membrane is partially detached from the cell wall, whereby protein in the periplasm easily leaks out to the outside of the outer membrane (i.e., to the outside of the bacterial cell). Thus, the modified cyanobacterium according to the present embodiment was found to have drastically improved secretory productivity of protein.

### (6) Identification of secreted intracellular metabolite

### (6-1) Sample preparation

20 µl of an aqueous solution containing an internal standard with a concentration adjusted to 1,000 µM was added to 80 µl of the culture supernatant of the modified cyanobacterium, and the mixture was stirred, ultrafiltered, and then subjected to measurement.

### (6-2) CE (capillary electrophoresis)-TOFMS (time-of-flight mass spectrometry) analysis

In this test, measurement in cationic and anionic modes was performed under the following conditions.

### [Cationic mode]

Apparatus : Agilent CE-TOFMS system
Capillary: Fused silica capillary i.d. 50 µm × 80 cm
Measurement conditions:
   Run buffer: Cation buffer solution (p/n: H3301-1001)
   CE voltage: Positive, 30 kV
   MS ionization: ESI positive
   MS scan range: m/z 50-1,000

### [Anionic mode]

Apparatus: Agilent CE-TOFMS system
Capillary: Fused silica capillary i.d. 50 µm × 80 cm
Measurement conditions:
   Run buffer: Anion buffer solution (p/n: H3301-1001)
   CE voltage: Positive, 30 kV
   MS ionization: ESI negative
   MS scan range: m/z 50-1,000

### (6-3) Data processing

Peaks with a signal/noise ratio of 3 or more were automatically detected as peaks detected in CE-TOFMS, using automatic integration software MasterHands(R) ver. 2.17.1.11. The detected peaks were checked against the values of all substances registered in the metabolite library of HMT (Human Metabolome Technologies Inc.), on the basis of the values of a mass-charge ratio (m/z) and a migration time inherent in each metabolite to search for metabolites contained in the culture supernatant of the modified cyanobacterium. Acceptable errors for search were +/-0.5 min in the migration time and +/-10 ppm in m/z. The concentration of each identified metabolite was calculated by single-point calibration of 100 µM. The identified major metabolites are described in Table 5.

**[Table 5]**

| | **Metabolite name** | **Concentration (*µ*M)** | |
|---|---|---|---|
| | | **slr1841-suppressed strain** | **slr0688-suppressed strain** |
| 1 | 3-hydroxybutyric acid | 1.3 | 1.2 |
| 2 | adenosine | 0.8 | 0.6 |
| 3 | aspartic acid | 4.6 | 3.0 |
| 4 | cytidine | 0.7 | 0.5 |
| 5 | guanos i ne | 1.5 | 1.1 |
| 6 | Gluconic acid | 2. 7 | 3.9 |
| 7 | Glutathione (GSSG) | 0.4 | 0.4 |
| 8 | Lactic acid | 3.2 | 4. 7 |
| 9 | Mal ic acid | 1.4 | 2. 8 |
| 10 | p-aminobeizoic acid | 1.7 | 0.9 |
| 11 | S-adenosylmethionine | 0.2 | 0.1 |
| 12 | spermidine | 4.2 | 3.0 |

All of these 12 types of intracellular metabolites were contained in each of the culture supernatants of the slr1841-suppressed strain of Example 1 and the slr0688-suppressed strain of Example 2. None of these metabolites were contained in the culture supernatant of the control strain of Comparative Example 1, though data is not described herein. From these results, it was able to be confirmed that in the modified strains of Example 1 and Example 2, the outer membrane is partially detached from the cell wall, whereby intracellular metabolites easily leak out to the outside of the outer membrane (i.e., to the outside of the bacterial cell).

### (7) Cultivation test and acidic invertase activation measurement test

Subsequently, in order to evaluate the plant acidic invertase activating effect of the secretion of the modified cyanobacterium (here, the culture supernatant of the modified cyanobacterium), the following plant cultivation tests were carried out. Specifically, a spinach cultivation test was carried out to evaluate its effect on leaf vegetable production. Also, a strawberry cultivation test was carried out to evaluate its effect on fruit production. Hereinafter, each of these cultivation tests will be described.

### (7-1) Spinach cultivation test

First, commercially available culture soil was placed in pots for cultivation (12 cm × 10 cm), and three seeds of spinach were sowed per pot. Cultivation was performed at a temperature of 23°C inside the room for 40 days under 10-hour light and 14-hour dark conditions using a white light source with a photon flux density of 200 µmol/m²/s. During this period, each pot was fed with 50 mL of distilled water every 2 days. Approximately 1 week after the start of cultivation, seedlings were thinned out at the cotyledonary stage to equalize individual sizes among the pots.

### (Example 3)

After equalization of individual sizes among the pots as described above, the culture supernatant of the modified cyanobacterium (hereinafter, referred to as the secretion of the modified cyanobacterium) was added at 5 mL per plant to the base of spinach once a week. After cultivation for 40 days and harvesting, the dry weight of shoot was measured, and a mean and standard deviation (SD) thereof were determined. Acidic invertase activity was measured by a method given below, and a mean and standard deviation (SD) thereof were determined. The modified cyanobacterium was the slr1841-suppressed strain of Example 1 and the slr0688-suppressed strain of Example 2.

### (Acidic invertase activity measurement)

Several leaves having a leaf length of approximately 10 cm were cut out of each plant, and their weights were measured. The leaves were frozen in liquid nitrogen and then ground in a mortar. 3 mL of an extraction buffer given below was added thereto, and the leaves were homogenized using a glass homogenizer to prepare extracts. 20 µL of the extracts was added to 180 µL of a solution for acidic invertase reaction (consisting of 50 mM sodium acetate pH 4.3 and 0.1 M sucrose), and left standing at 30°C for 1 hour so that the extracts were reacted with the acidic invertase reaction solution. Then, the reaction was terminated by incubation at 85°C for 3 minutes. The amount of glucose formed during this reaction was quantified using a commercially available glucose quantification kit. From this value, the amount of glucose formed by 1 g (weight) of the leaves in 1 hour was calculated as acidic invertase activity, and a mean and standard deviation (SD) were determined.

### <Extraction buffer>

The extraction buffer had the following composition.

100 mM HEPES (4-(2-hydroxyethyl)-1-piperazineethanesulfonic acid)-KOH pH7.4
5 mM MgCl₂
1 mM EDTA (ethylenediaminetetraacetic acid)
1 mM EGTA (ethyleneglycol-bis(β-aminoehylether)-tetraacetic acid)
1 mM PMSF (phenmethylsulphonyl-fluoride)
5 mM DTT (dithiothreitol)
1 mL/L Triton X-100
200 mL/L glycerol
5 mM thiourea

### (Comparative Example 2)

The operation was performed in the same manner as in Example 3 except that water was used instead of the secretion of the modified cyanobacterium.

### (Results)

The results of Example 3 and Comparative Example 2 are shown in FIG. 10 and FIG. 11. FIG. 10 is a graph illustrating a mean of the acidic invertase activity of spinach cultivated in Example 3 and Comparative Example 2. FIG. 11 is a graph illustrating a mean of the dry weight of shoot per plant (referred to as an average plant weight) of spinach cultivated in Example 3 and Comparative Example 2.

As illustrated in FIG. 10, the acidic invertase activity of spinach cultivated in Example 3 was elevated by approximately 2.3 times as compared with Comparative Example 2.

As illustrated in FIG. 11, the plant weight of spinach cultivated in Example 3 was increased by approximately 1.4 times as compared with Comparative Example 2.

It was thus confirmed that the application of the secretion of the modified cyanobacterium to spinach activates the acidic invertase of spinach and the promotion of growth and weight gain of spinach are observed by the activation of acidic invertase.

### (7-2) Strawberry cultivation test

Approximately 7 cm long strawberry seedlings having approximately 9 leaves were settled-planted in pots for cultivation (12 cm × 10 cm) containing commercially available culture soil. Cultivation was performed at temperatures of 20°C for the light period and 15°C for the dark period for 150 days under 14-hour light and 10-hour dark conditions using a white light source with a photon flux density of 200 µmol/m²/s. During this period, each pot was fed with 50 mL of distilled water every alternate day. Also, a commercially available chemical fertilizer (500-fold dilution of a stock solution containing 6% of total nitrogen, 10% of water-soluble phosphoric acid, 5% of water-soluble potassium, 0.05% of water-soluble magnesium, 0.001% of water-soluble manganese, and 0.005% of water-soluble boron) was applied at 100 mL per pot once 50 days.

### (Example 4)

During the cultivation period described above, the secretion of the modified cyanobacterium was added at 5 mL per plant to the base of the plant once a week. Strawberry whose fruit ripened and turned red was harvested in order, and the number of harvested fruits was recorded. The weights and sugar contents (Brix values) of the harvested fruits were measured, and a mean and standard deviation (SD) thereof were determined. Acidic invertase activity was measured in the same manner as in Example 3 except that several fruits were used. Here, the amount of glucose formed by 1 g of the fruits in 1 hour was regarded as acidic invertase activity. The modified cyanobacterium was the slr1841-suppressed strain of Example 1 and the slr0688-suppressed strain of Example 2.

### (Comparative Example 3)

The operation was performed in the same manner as in Example 4 except that water was used instead of the secretion of the modified cyanobacterium.

### (Results)

The results of Example 4 and Comparative Example 3 are shown in FIG. 12 to FIG. 16. FIG. 12 is a graph illustrating a mean of the acidic invertase activity of strawberry cultivated in Example 4 and Comparative Example 3. FIG. 13 is a graph illustrating an average number of fruits per plant of strawberry cultivated in Example 4 and Comparative Example 3. FIG. 14 is a graph illustrating an average fruit weight per plant of strawberry cultivated in Example 4 and Comparative Example 3. FIG. 15 is a graph illustrating an average sugar content per plant of strawberry cultivated in Example 4 and Comparative Example 3.

FIG. 16 provides photographs of typical fruits in order to visualize the states of respective fruits in Example 4 and Comparative Example 3.

As illustrated in FIG. 12, the plant acidic invertase activity of strawberry cultivated in Example 4 was elevated by approximately 2.3 times as compared with Comparative Example 3.

As illustrated in FIG. 13, the average number of fruits per plant harvested in Example 4 was increased by approximately 1.4 times as compared with Comparative Example 3.

As illustrated in FIG. 14, the average fruit weight of strawberry harvested in Example 4 had no significant difference. In short, the average fruit weight of strawberry cultivated in Example 4 was equivalent to that of Comparative Example 3, despite the fact that the number of fruits harvested per plant was larger in Example 4.

As illustrated in FIG. 15, the average sugar content (Brix sugar content) of strawberry harvested in Example 4 was improved by approximately 1.1 times as compared with Comparative Example 3. In short, the average sugar content of fruits of strawberry cultivated in Example 4 was higher than that of Comparative Example 3, despite the fact that the number of fruits harvested was larger in Example 4.

As illustrated in FIG. 16, the harvested fruits of strawberry did not differ in appearance such as size, shape, and color between Example 4 and Comparative Example 3. In short, the fruit size and the like of strawberry cultivated in Example 4 were equivalent to those of Comparative Example 3, despite the fact that the number of fruits harvested was larger in Example 4.

It was thus confirmed that the application of the secretion of the modified cyanobacterium to strawberry activates the acidic invertase of strawberry and effects such as the promotion of growth, increase in the number of fruits, maintenance of fruit size, and elevation in fruit sugar content are observed by the activation of acidic invertase.

### (Conclusion)

From the results of the cultivation tests of spinach and strawberry and the acidic invertase activity measurement, the plant acidic invertase activator according to the present embodiment was able to be confirmed to have effects such as promotion of growth, increase in yield, weight gain, and elevation in fruit sugar content on a plurality of crop species.

### [Industrial Applicability]

The present disclosure can provide a modified cyanobacterium having improved secretory productivity of a plant acidic invertase activating substance. The substance can be efficiently produced by culturing the modified cyanobacterium of the present disclosure. For example, the addition of the substance to soil can cause plant acidic invertase activation, and thus crop production can be promoted.

### [Reference Signs List]

- 1: inner membrane
- 2: peptidoglycan
- 3: sugar chain
- 4: cell wall
- 5: outer membrane
- 6: SLH domain-containing outer membrane protein
- 7: SLH domain
- 8: organic channel protein
- 9: cell wall-pyruvic acid modifying enzyme

## Claims

1. A plant acidic invertase activator production method comprising:
preparing a modified cyanobacterium in which a function of a protein involved in binding between an outer membrane and a cell wall of cyanobacterium is suppressed or lost; and
causing the modified cyanobacteria to secrete a secretion involved in activating an acidic invertase of a plant.

2. The plant acidic invertase activator production method according to claim 1, wherein
the protein involved in the binding between the outer membrane and the cell wall is at least one of a surface layer homology (SLH) domain-containing outer membrane protein or a cell wall-pyruvic acid modifying enzyme.

3. The plant acidic invertase activator production method according to claim 2, wherein
the SLH domain-containing outer membrane protein is:
Slr1841 having an amino acid sequence represented by SEQ ID NO: 1;
NIES970_09470 having an amino acid sequence represented by SEQ ID NO: 2;
Anacy_3458 having an amino acid sequence represented by SEQ ID NO: 3; or
a protein having an amino acid sequence that is at least 50 percent identical to the amino acid sequence of any one of the Slr1841, the NIES970_09470, and the Anacy_3458.

4. The plant acidic invertase activator production method according to claim 2, wherein
the cell wall-pyruvic acid modifying enzyme is:
Slr0688 having an amino acid sequence represented by SEQ ID NO: 4;
Synpcc7942_1529 having an amino acid sequence represented by SEQ ID NO: 5;
Anacy_1623 having an amino acid sequence represented by SEQ ID NO: 6; or
a protein having an amino acid sequence that is at least 50 percent identical to the amino acid sequence of any one of the Slr0688, the Synpcc7942_1529, and the Anacy_1623.

5. The plant acidic invertase activator production method according to claim 1, wherein
a gene which causes expression of the protein involved in the binding between the outer membrane and the cell wall is deleted or inactivated.

6. The plant acidic invertase activator production method according to claim 5, wherein
the gene which causes expression of the protein involved in the binding between the outer membrane and the cell wall is at least one of a gene encoding an SLH domain-containing outer membrane protein or a gene encoding a cell wall-pyruvic acid modifying enzyme.

7. The plant acidic invertase activator production method according to claim 6, wherein
the gene encoding the SLH domain-containing outer membrane protein is:
slr1841 having a nucleotide sequence represented by SEQ ID NO: 7;
nies970_09470 having a nucleotide sequence represented by SEQ ID NO: 8;
anacy_3458 having a nucleotide sequence represented by SEQ ID NO: 9; or
a gene having a nucleotide sequence that is at least 50 percent identical to the nucleotide sequence of any one of the slr1841, the nies970_09470, and the anacy_3458.

8. The plant acidic invertase activator production method according to claim 6, wherein
the gene encoding the cell wall-pyruvic acid modifying enzyme is:
slr0688 having a nucleotide sequence represented by SEQ ID NO: 10;
synpcc7942_1529 having a nucleotide sequence represented by SEQ ID NO: 11;
anacy_1623 having a nucleotide sequence represented by SEQ ID NO: 12; or
a gene having a nucleotide sequence that is at least 50 percent identical to the nucleotide sequence of any one of the slr0688, the synpcc7942_1529, and the anacy_1623.

9. A plant acidic invertase activator comprising:
a secretion of a modified cyanobacterium in which a function of a protein involved in binding between an outer membrane and a cell wall of cyanobacterium is suppressed or lost.

10. A plant acidic invertase activation method comprising:
using the plant acidic invertase activator according to claim 9 on a plant.
